# EUROPÄISCHE PATENTSCHRIFT

(11) **EP 2 364 186 B1**
(45) Veröffentlichungstag und Bekanntmachung des Hinweises auf die Patenterteilung: **25.12.2013**
(21) Anmeldenummer: 09740085.7
(22) Anmeldetag: 09.10.2009
(51) Int. Cl.: A61Q 13/00, C07C 49/76, C11B 9/00, C11D 3/50, A61K 8/35, A61Q 19/10, C07C 49/82, C07C 49/83

(54) **PHOTOLABILE DUFTSPEICHERSTOFFE**
PHOTOLABILE FRAGRANCE STORAGE SUBSTANCES
SUBSTANCES PHOTOLABILES ACCUMULATRICES DE PARFUM

(30) Priorität: 09.12.2008 DE 102008060886
(43) Veröffentlichungstag der Anmeldung: 14.09.2011
(73) Patentinhaber: Henkel AG & Co. KGaA, 40589 Düsseldorf (DE)
(72) Erfinder: HUCHEL, Ursula, 50670 Köln (DE); KROPF, Christian, 40724 Hilden (DE); GRIESBECK, Axel, 50937 Köln (DE); HINZE, Olga, 50679 Köln (DE); SUNDERMEIER, Uta, 40225 Düsseldorf (DE)
(86) Internationale Anmeldenummer: PCT/EP2009/063145
(87) Internationale Veröffentlichungsnummer: WO 2010/066486

(56) Entgegenhaltungen:
- WO-A1-2005/051336
- DE-A1- 2 445 649
- US-A1- 2002 077 508
- US-A1- 2002 094 938
- US-A1- 2003 129 212
- HASEGAWA E ET AL: "Photoinduced Electron Transfer Reactions of alpha,beta-Epoxy Ketones with 2-Phenyl-N,N-dimethylbenzimidazoline (PDMBI): Significant Water Effect on the Reaction Pathway" TETRAHEDRON LETTERS, ELSEVIER, AMSTERDAM, NL LNKD- DOI:10.1016/0040-4039(96)01578-X, Bd. 37, Nr. 39, 23. September 1996 (1996-09-23), Seiten 7079-7082, XP004030831 ISSN: 0040-4039
- ABATE, AGNESE ET AL.: "CHIRALITY AND FRAGRANCE CHEMISTRY: STEREOISOMERS OF THE COMMERCIAL CHIRAL ODORANTS MUGUESIA AND PAMPLEFLEUR" JOURNAL OF ORGANIC CHEMISTRY, Bd. 2005, Nr. 4, 26. Januar 2005 (2005-01-26), Seiten 1281-1290, XP002586731 WASHINGTON, US ISSN: 0022-3263 DOI: 10.1021/jo048445j

## Beschreibung

Die vorliegende Erfindung betrifft Wasch- oder Reinigungsmittel, welche mindestens ein bestimmtes β-Hydroxyketon umfassen, welches insbesondere bei Einwirken elektromagnetischer Strahlung eine Carbonylverbindung freisetzen kann, wie vorzugsweise einen Riechstoffaldehyd oder ein Riechstoffketon. Ferner betrifft sie ein Verfahren zur Freisetzung von Riechstoffaldehyden bzw. Riechstoffketonen aus bestimmten β-Hydroxyketonen. Sie betrifft ferner ein Verfahren zur lang anhaltenden Beduftung von Oberflächen. Sie betrifft die Verwendung von bestimmten β-Hydroxyketonen als Duftstoffvorläufer in Wasch- und Reinigungsmitteln, in kosmetischen Mitteln sowie in Lufterfrischern.

Textil- und Oberflächenbehandlungsmittel bzw. kosmetische Mittel enthalten zumeist Duftstoffe (Riechstoffe), die den Mitteln einen angenehmen und frischen Geruch verleihen. Die Begriffe Riechstoffe und Duftstoffe werden in dieser Erfindung synonym gebraucht. Die Duftstoffe maskieren dabei zumeist die Eigenduftnote der anderen Inhaltstoffe, so dass beim Verbraucher ein positiver Geruchseindruck entsteht. Im Bereich Waschmittel sind Duftstoffe besonders wichtige Bestandteile der Zusammensetzung, da die Wäsche sowohl im feuchten als auch im trockenen Zustand einen angenehmen und frischen Duft aufweisen soll. Ein grundsätzliches Problem bei der Verwendung von Duftstoffen besteht jeweils darin, dass es sich bei diesen um flüchtige Substanzen handelt, ansonsten könnte kein Dufteffekt erzielt werden. Jedoch strebt man andererseits einen möglichst langanhaltenden und gleichbleibenden Dufteffekt an. Bekannt ist z.B., dass sich der Dufteindruck eines Parfums im Laufe der Zeit verändert, weil die Riechstoffe, die die frischen und leichten Noten des Parfüms darstellen durch ihren hohen Dampfdruck schneller abdampfen als die Duftstoffe, die die Herz- und Basisnoten darstellen.

Ein Ansatz zur Lösung dieses Problems, nämlich einen möglichst langanhaltenden und gleichbleibenden Dufteffekt zu erzielen, besteht darin, Duftstoffe auf Trägermaterialien aufzubringen und die bedufteten Träger zu beschichten, oder Duftstoffe zu verkapseln oder in Verbindungen einzulagern (beispielsweise Cyclodextrin-Parfüm-Komplexe). Des Weiteren existiert die Möglichkeit, die Duftstoffe chemisch an Trägermedien zu binden, wobei die chemische Bindung langsam gespalten und der Duftstoff hierdurch freigesetzt wird. Eine solche Träger-gebundene Vorform eines Duftstoffes wird auch als "Pro-Fragrance" oder Duftspeicherstoff bezeichnet. In diesem Zusammenhang offenbart die internationale Patentanmeldung WO2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird. Der beschriebene Prozess muss dabei für eine wirksame Freisetzung des Duftstoffs die Anwesenheit von Sauerstoff und Wasser tolerieren.

In diesem Zusammenhang offenbart das US-Patent 6,949,680 die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität die durch den photochemisch induzierten Zerfall erst verzögert und über einen längeren Zeitraum hinweg auf einer bestimmten Oberfläche freigesetzt wird. Dabei werden die genannten photolabilen Phenyl- oder Pyridylketone als Duftspeicherstoffe in einem aufwendigen, vielstufigen Syntheseverfahren unter Einsatz von Schutzgruppenoperationen hergestellt, wobei die Synthese für jeden einzelnen Aktivstoff individuell angepasst werden muss.

Das Ziel der vorliegenden Erfindung war es, eine verzögerte Freisetzung von Duftaldehyden (Riechstoffaldehyde) und Duftketonen (Riechstoffketone) zu ermöglichen.

Überraschenderweise wurde nun gefunden, dass eine Zusammensetzung, umfassend bestimmte, nachfolgend beschriebene β-Hydroxyketone, die verzögerte Freisetzung von Duftstoffaldehyden (Riechstoffaldehyden) oder Duftstoffketonen (Riechstoffketonen) erlaubt.

Gegenstand der vorliegenden Erfindung ist daher ein Wasch- oder Reinigungsmittel, enthaltend mindestens ein β-Hydroxyketon der allgemeinen Formel (I) 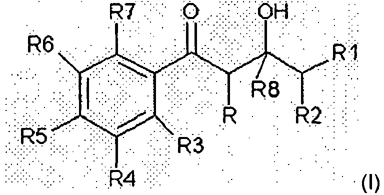 wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht, und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono- oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen,
dadurch gekennzeichnet, dass das genannte β-Hydroxyketon der allgemeinen Formel (I) bei Einwirken elektromagnetischer Strahlung, insbesondere umfassend die Wellenlängen von 200 bis 400 nm, zumindest ein Riechstoffaldehyd oder ein Riechstoffketon freisetzt.

Die Erfindung ermöglicht auch die Verlängerung der Duftwirkung von anderen Duftstoffen, welche ebenfalls in der Zusammensetzung enthalten sind. Sie ermöglicht ferner die Erzielung eines lange anhaltenden Frischegeruches. Sie ermöglicht die lange anhaltende Beduftung von Oberflächen. Sie ermöglicht eine steuerbare, photoinduzierte Freisetzung von Riechstoffaldehyden sowie Riechstoffketonen.

Erfindungsgemäß bevorzugte Wasch- oder Reinigungsmittel enthalten mindestens ein β-Hydroxyketon der allgemeinen Formel (II) und/oder der allgemeinen Formel (III), 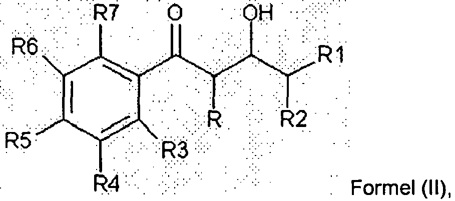 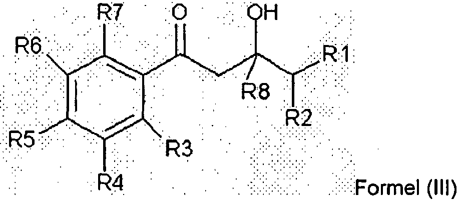 wobei in diesen Formeln (II) und (III), unabhängig voneinander, die Reste R, R1, R2, R3, R4, R5, R6, R7, R8 definiert sind wie im Zusammenhang mit der Formel (I) zuvor bereits angegeben. Dabei ist es bezogen auf die Formel (II) bevorzugt, wenn der Rest R für eine lineare oder verzweigte, substituierte oder unsubstituierte Alkyl- oder Alkylengruppe von C₁ bis C₂₀ steht, vorzugsweise aber für einen Methylrest steht. Bezogen auf die Formel (III) ist es bevorzugt, wenn der Rest R8 für eine lineare oder verzweigte, substituierte oder unsubstituierte Alkyl- oder Alkylengruppe von C₁ bis C₂₀ steht, vorzugsweise aber für einen Methylrest steht. Die Formeln (II) und (III) bilden einfache Untermengen der Formel (I) ab.

Weiterhin bevorzugt ist eine erfindungsgemäße Verbindung der allgemeinen Formel (I) in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 vorzugsweise für ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Erfindung steht R5 für -Cl, -Br, -NO₂ oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe. Eine Substitution in para-Position (R5) ist besonders bevorzugt, da die elektronische Struktur des aromatischen Rings hier am effektivsten modifiziert werden kann, wodurch das Absorptionsmaximum von Verbindungen der allgemeinen Formel (I) leicht an eine bestimmte Wellenlänge angepasst werden kann. Bevorzugt ist ebenfalls eine erfindungsgemäße Verbindung der allgemeinen Formel (I), in der alle fünf Arylsubstituenten R3, R4, R5, R6 und R7 für Wasserstoff stehen. Das zuvor Gesagte bezüglich der allgemeinen Formel (I) und den fünf Arylsubstituenten R3, R4, R5, R6 und R7 trifft selbstverständlich ebenso auf die allgemeinen Formel (II) sowie (III) zu.

Bei Einwirken elektromagnetischer Strahlung, insbesondere umfassend die Wellenlängen von 200 bis 400 nm, kann das genannte β-Hydroxyketon der allgemeinen Formel (I) erfindungsgemäß eine vorzugsweise wohlriechende Carbonylverbindung, also ein Duftstoffketon oder-aldehyd freisetzen. Als elektromagnetische Strahlung im Sinne der vorliegenden Erfindung kann vorzugsweise das natürliche Sonnenlicht dienen. Dies trifft selbstverständlich auch für die β-Hydroxyketone der allgemeinen Formel (II) sowie (III) zu.

In einer bevorzugten Ausführungsform der Erfindung zeichnet sich ein erfindungsgemäßes Wasch-oder Reinigungsmittel dadurch aus, dass das β-Hydroxyketon bei Einwirken elektromagnetischer Strahlung umfassend die Wellenlängen von 200 bis 400 nm eine Verbindung der Formel (IV) 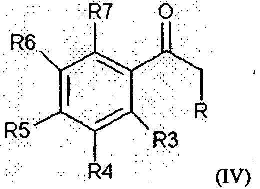 sowie eine Carbonylverbindung der Formel (V) freisetzt 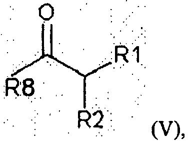 wobei die Reste R, R1, R2, R3, R4, R5, R6, R7, R8 definiert sind wie zuvor im Zusammenhang mit der Formel (I) bereits angegeben.
Dabei ist es bevorzugt, wenn in der Formel (IV) der Rest R für ein Wasserstoffatom oder für einen Methylrest steht. Dabei ist es ebenfalls bevorzugt, wenn in der Formel (V) der Rest R8 für ein Wasserstoffatom oder für einen Methylrest steht.

Die vorliegende Erfindung ermöglicht somit die gezielte Freisetzung von wohlriechenden Aldehyden sowie Ketonen durch das Einwirken elektromagnetischer Strahlung, insbesondere umfassend die Wellenlängen von 200 bis 400 nm.

Wenn durch das erfindungsgemäße Wasch- oder Reinigungsmittel bei dem Einwirken elektromagnetischer Strahlung umfassend die Wellenlängen von 200 bis 400 nm zumindest ein Riechstoffaldehyd freigesetzt wird, vorzugsweise ausgewählt aus der Gruppe umfassend Melonal, Triplal, Ligustral, Adoxal; Anisaldehyd; Cymal; Ethylvanillin; Florhydral; Helional; Heliotropin; Hydroxycitronellal; Koavon; Laurinaldehyd; Lyral; Methyl-nonyl-acetaldehyd; p,t-Bucinal; Phenylacetaldehyd; Undecylenaldehyd; Vanillin;2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzyaldehyd, 1,2,3,4,5,6,7,8-octahydro-8,8- Dimethyl-2-naphthaldehyd, 2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2.1.0 (2,6)]-decylidene-8)-butanal; Octahydro-J-methano-1H-indenecarboxaldehyd; 3-Ethoxy-4-hydroxybenzaldehyd, para-Ethyl-alpha, alpha-dimethylhydrozimtaldehyd, alpha-Methyl-3,4-(methylenedioxy)-hydrozimtaldehyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyl octanal, Undecenal, 2,4,6-Trimethyl-3-cyclohexene-1-carboxaldehyd, 4-(3)(4-Methyl-3-pentenyl)-3-cyclohexen-carboxaldehyd, 1-Dodecanal, 2,4-Dimethyl cyclohexene-S-carboxaldehyd, 4-(4-Hydroxy-4-methyl pentyl-S-cylohexene-1-carboxaldehyd, 7-Methoxy-3,7-dimethyloctan-1-al, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd,1-methyl-4-(4-methyl-3-pentenyl)-3-cyclohexene-1-carboxaldehyd, 5 oder 6 Methoxyhexahydro-4,7-methanoindan-1 oder 2-carboxyaldehyd; 3,7-Dimethyloctan-1-al, 1 -Undecanal, 10-Undecen-1-al, 4-Hydroxy-3-methoxybenzaldehyd, 1-Methyl-3-(4-methylpentyl)-3-cyclohexencarboxyaldehyd, 7-Hydroxy-3,7-dimethyl-octanal; trans-4-decenal, 2,6-Nonadienal, para-Tolylacetaldehyd; 4-Methylphenylacetaldehyd, 2-Methyl-4-(2,6,6-trimethyl-1-cyclohexen-1-yl)-2-butenal, ortho-Methoxyzimtaldehyd, S.S.δ-Trimethyl-S-cyclohexenecarboxaldehyd; 3,7-Dimethyl-2-methylene-6-octenal, Phenoxyacetaldehyd; 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha-Methyl-4-(I-methylethyl) benzeneacetaldehyd, 6,6-Dimethyl-2-norpinene-2-propionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9-Decenal, 3-Methyl-5-phenyl-1-pentanal, Methylnonyl acetaldehyd, 1-p-Menthene-q-carboxaldehyd, Citral, Lilial, 1-Decanal, Florhydral , 2,4-Dimethyl-3-cyclohexen-1-carboxaldehyd oder Gemische davon, so liegt eine bevorzugte Ausführungsform der Erfindung vor.

Ebenso liegt eine weitere bevorzugte Ausführungsform der Erfindung vor, wenn bei Einwirken elektromagnetischer Strahlung umfassend die Wellenlängen von 200 bis 400 nm aus dem erfindungsgemäßen Wasch- oder Reinigungsmittel zumindest ein Riechstoffketon freigesetzt wird, vorzugsweise ausgewählt aus der Gruppe umfassend Buccoxim; Iso jasmon; Methyl beta naphthyl keton; Moschus indanon; Tonalid/Moschus plus; Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro- Beta-Ionon, Gamma-Methyl so genanntes Ionon, Fleuramon, Dihydrojasmon, Cis-Jasmon, Iso-E- Super, Methyl-cedrenyl-keton oder Methyl-cedrylon, Acetophenon, Methyl-acetophenon, Para- Methoxy-acetophenon, Methyl-beta-naphtyl-keton, Benzyl-aceton, Benzophenon, Para-Hydroxy- phenyl-butanon, Celery Keton oder Livescon, 6-Isopropyldecahydro-2-naphton, Dimethyl- octenon, Freskomenth, 4-(1-Ethoxyvinyl)-3,3,5,5,-tetramethyl-cyclohexanon, Methyl-heptenon, 2-(2-(4- Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl- cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran sowie Hedion.

Die erfindungsgemäß freisetzbaren Riechstoffaldehyde und Riechstoffketone können eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen.

Eine weitere bevorzugte Ausführungsform der Erfindung liegt vor, wenn zumindest eines der folgenden β-Hydroxyketone gemäß den Formeln (VI) oder (VII) enthalten ist:

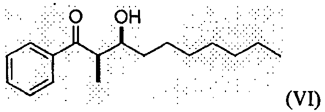

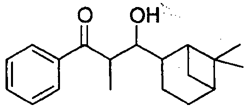

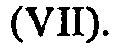

Das erfindungsgemäße Wasch- oder Reinigungsmittel enthält β-Hydroxyketone der allgemeinen Formel (I) vorzugsweise in Mengen zwischen 0,0001 und 95 Gew.-%, bezogen auf das gesamte Wasch- oder Reinigungsmittel. Eine mögliche Obergrenze für die β-Hydroxyketone in dem Wasch-oder Reinigungsmittel kann z.B auch bei 80 Gew.-%, 70 Gew.-%, 60 Gew.-%, 50 Gew.-%, 40 Gew.-%, 30 Gew.-%, 20 Gew.-%, 10 Gew.-% oder z.B. auch bei 5 Gew.-% liegen, bezogen auf das gesamte Wasch- oder Reinigungsmittel. Ein mögliche Untergrenze für die β-Hydroxyketone in dem Wasch- oder Reinigungsmittel kann z.B auch bei 0,001 Gew.-%, 0,01 Gew.-%, 0,1 Gew.-% oder z.B. 0,5 Gew.-% liegen, bezogen auf das gesamte Wasch- oder Reinigungsmittel.

Das erfindungsgemäße Wasch- oder Reinigungsmittel kann neben den β-Hydroxyketonen weitere Inhaltsstoffe umfassen, wie z.B. Lösungsmittel, Trägermaterialen, weitere Duftstoffe, Farbstoffe, Tenside usw. Die Art und Menge der zusätzlich enthaltenen Inhaltsstoffe ist prinzipiell unkritisch und richtet sich primär nach dem Einsatzzweck der erfindungsgemäßen Wasch- oder Reinigungsmittel. Ein Waschmittel enthält sinnvollerweise auch für Waschmittel übliche Inhaltsstoffe wie z.B. Tenside, Builder usw.

In einer bevorzugten Ausführungsform der Erfindung enthält das erfindungsgemäße Wasch- oder Reinigungsmittel mindestens einen zusätzlichen Duftstoff. Mit "zusätzlichem" Duftstoff sind also andere Substanzen als die ohnehin enthaltenen β-Hydroxyketone der allgemeinen Formel (I) gemeint, welche eine Duftwirkung entfalten können.

Die bevorzugt zusätzlich einsetzbaren Duftstoffe bzw. Parfümöle sind keinen Beschränkungen unterworfen. So können insbesondere synthetische oder natürliche Riechstoffverbindungen vom Typ der Ester, Ether, Aldehyde (Duftaldehyde, Riechstoffaldehyde), Ketone (Duftketone, Riechstoffketone), Alkohole, Kohlenwasserstoffe, Säuren, Kohlensäureester, aromatischen Kohlenwasserstoffe, aliphatischen Kohlenwasserstoffe, gesättigten und / oder ungesättigten Kohlenwasserstoffe und Mischungen daraus als Duftstoffe verwendet werden.

Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann bekannt. Die Duftketone können alle Ketone umfassen, die einen erwünschten Duft oder ein Frischeempfinden verleihen können, z.B. diejenigen welche in der Beschreibung bereits genannt wurden. Es können auch Gemische unterschiedlicher Ketone eingesetzt werden. Bevorzugt können die Ketone ausgewählt sein aus Alpha Damascon, Delta Damascon, Iso Damascon, Carvon, Gamma-Methylionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise die bereits vorher im Text genannten Aldehyde.

Die Duftaldehyde und Duftketone können eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen.

Für weitere geeignete Duftstoffe, ausgewählt aus Aldehyden und Ketonen, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen.

Geeignete Duftstoffe vom Typ der Ester sind beispielsweise Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Riechstoffverbindungen vom Typ der Kohlenwasserstoffe sind z.B. Terpene wie Limonen und Pinen.

Geeignete Duftstoffe vom Typ Ether sind beispielsweise Benzylethylether und Ambroxan. Geeignete Duftsstoffalkohole sind z.B. 10-Undecen-1-ol, 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenylpentanol, 3-Octanol, 1-Octen-3-ol, 3-Phenylpropanol, 4-Heptenol, 4-Isopropylcyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Anethol, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadienol, trans-2-Nonen-1-ol, trans-2-Octenol, Undecanol, Vanillin, Zimtalkohol, wobei, wenn mehrere Duftstoffalkohole vorhanden sind, diese unabhängig voneinander ausgewählt sein können.

Duftstoffe bzw. Parfümöle können auch natürliche Riechstoffgemische sein, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl. Bei den Duftstoffen bzw. Parfümölen kann es sich auch um ätherische Öle, wie z.B. Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl handeln.

Die Gesamtmenge der einsetzbaren zusätzlichen Duftstoffe in dem erfindungsgemäßen Wasch-oder Reinigungsmittel beträgt vorzugsweise zwischen 0,01 und 5 Gew.-%, besonders vorzugsweise zwischen 0,1 und 3 Gew.-% sowie ganz besonders bevorzugt zwischen 0,5 und 2 Gew.-% bezogen auf die Gesamtmenge des Wasch- oder Reinigungsmittels. Bevorzugt werden Mischungen verschiedener Duftstoffe (aus den verschiedenen oben genannten Duftstoffklassen) verwendet, die gemeinsam eine ansprechende Duftnote erzeugen.

Bei dem Wasch- oder Reinigungsmittel kann es sich insbesondere um ein Textilbehandlungsmittel in Form eines Textilwaschmittels, Weichspülers, weichmachenden Waschmittels oder Waschhilfsmittels handeln. Ebenso kann es sich z.B. um ein Reinigungsmittel für harte Oberflächen handeln, wie vorzugsweise um ein Geschirrspülmittel, insbesondere um ein maschinelles Geschirrspülmittel. Ebenso kann es sich um Reinigungsmittel wie z.B. Haushaltsreiniger, Allzweckreiniger, Fensterreiniger, Fußbodenreiniger usw. handeln.

Vorzugsweise ist das β-Hydroxyketon der allgemeinen Formel (I) in dem erfindungsgemäßen Wasch- oder Reinigungsmittel, insbesondere in Form eines Waschmittels, Weichspülers, weichmachenden Waschmittels oder Waschhilfsmittels, in Mengen von vorzugsweise zwischen 0,01 und 5 Gew.-%, besonders vorzugsweise zwischen 0,1 und 3 Gew.-% sowie ganz besonders bevorzugt zwischen 0,5 und 2 Gew.-%, jeweils bezogen auf die Gesamtmenge des Wasch- oder Reinigungsmittels, enthalten.

Ein erfindungsgemäßes Wasch- oder Reinigungsmittel kann fest oder flüssig sein, wobei flüssige Mittel bevorzugt sein können.

Insbesondere für den Fall, dass das erfindungsgemäße Mittel ein Wasch- oder Reinigungsmittel ist, ist es bevorzugt, dass es mindestens ein Tensid ausgewählt aus der Gruppe bestehend aus anionischen, nichtionischen, zwitterionischen und amphoteren Tensiden enthält.

Für den Fall, dass das erfindungsgemäße Mittel ein weichmachendes Waschmittel ("2in1") ist, ist es bevorzugt, dass es eine weichmachende Komponente sowie mindestens ein Tensid ausgewählt aus der Gruppe bestehend aus anionischen, nichtionischen, zwitterionischen und amphoteren Tensiden enthält.

Waschhilfsmittel werden zur gezielten Vorbehandlung der Wäsche vor dem Waschen bei Flecken oder starker Verschmutzung eingesetzt. Zu den Waschhilfsmitteln gehören beispielsweise Vorbehandlungsmittel, Einweichmittel, Entfärber und Fleckensalz.

Für den Fall, dass das erfindungsgemäße Mittel ein Weichspüler ist, ist es bevorzugt, dass es eine weichmachende Komponente enthält.

Weichspüler sind als erfindungsgemäße Mittel bevorzugt, da sie erst im letzten Schritt eines konventionellen Textilwaschvorgangs, dem Spülgang, in Kontakt mit den Textilien kommen und somit eine besonders große Menge der Duftstoffe auf das Textil aufziehen kann, ohne dass die Gefahr besteht, dass die Duftstoffe bei anschließenden Schritten wieder entfernt werden.

Ein weiterer Gegenstand der vorliegenden Erfindung ist ein Verfahren zur Freisetzungen von Carbonylverbindungen, vorzugsweise Riechstoffaldehyden und/oder-ketonen, der Formel (V) 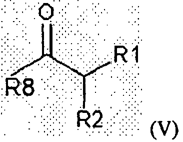 aus β-Hydroxyketonen der allgemeinen (Formel I) 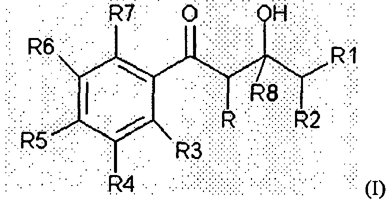 durch Einsatz elektromagnetischer Strahlung umfassend die Wellenlängen von 200 bis 400 nm, wobei die Reste R, R1, R2, R3, R4, R5, R6, R7, R8 wiederum so definiert sind wie zuvor bereits angegeben, insbesondere bei Anwendungen im Zusammenhang mit dem Einsatz von Wasch- oder Reinigungsmitteln.

In einer bevorzugten Ausführungsform des Verfahrens dient es zur Freisetzung von Riechstoffaldehyden, insbesondere ausgewählt aus der Gruppe umfassend Melonal, Triplal, Ligustral, Adoxal; Anisaldehyd; Cymal; Ethylvanillin; Florhydral; Helional; Heliotropin; Hydroxycitronellal; Koavon; Laurinaldehyd; Lyral; Methyl-nonyl-acetaldehyd; p,t-Bucinal; Phenylacetaldehyd; Undecylenaldehyd; Vanillin;2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd, 2,4-Dimethyl-3-cyclohexen-1-carboxyaldehyd, 2-Methyl-3- (isopropylphenyl)propanal, Decyl aldehyd, 2,6-Dimethyl-5-heptenal; 4-(tricyclo[5.2.1.0 (2,6)]- decylidene-8)-butanal; Octahydro-J-methano-IH-indenecarboxaldehyd; 3-Ethoxy-4-hydroxybenzaldehyd, para-Ethyl-alpha, alpha-dimethylhydrozimtaldehyd, alpha-Methyl-3,4- (methylenedioxy)-hydrozimtalde-hyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyl octanal, Undecenal, 2,4,6-Trimethyl-3-cyclohexene-1-carboxaldehyd, 4-(3)(4-Methyl-3-pentenyl)-3- cyclohexen-carboxaldehyd, 1-Dodecanal, 2,4-Dimethyl cyclohexene-S-carboxaldehyd, 4-(4- Hydroxy-4-methyl pentyl-S-cylohexene-1-carboxaldehyd, 7-Methoxy-3,7-dimethyloctan-1-al, 2- Methyl undecanal, 2-Methyl decanal, 1-nonanal, 1-Octanal, 2,6,10-Trimethyl-5,9-undecadienal, 2- Methyl-3-(4-tertbutyl)propanal, Dihydrozimtaldehyd,1-methyl-4-(4-methyl-3-pentenyl)-3- cyclohexene-1-carboxaldehyd, 5 oder 6 Methoxyhexahydro-4,7-methanoindan-1 oder 2- carboxyaldehyd; 3,7-Dimethyloctan-1-al, 1 -Undecanal, 10-Undecen-1-al, 4-Hydroxy-3-methoxybenzaldehyd, 1-Methyl-3-(4-methylpentyl)-3-cyclohexencarboxyaldehyd, 7-Hydroxy-3,7-dimethyl-octanal; trans-4-decenal, 2,6-Nonadienal, para-Tolylacetaldehyd; 4-Methylphenylacetaldehyd, 2-Methyl-4-(2,6,6-trimethyl-1-cyclohexen-1-yl)-2-butenal, ortho- Methoxyzimtaldehyd, S.S.δ-Trimethyl-S-cyclohexenecarboxaldehyd; 3,7-Dimethyl-2-methylene-6- octenal, Phenoxyacetaldehyd; 5,9-Dimethyl-4,8-decadienal, Peony aldehyd (6,10-dimethyl-3-oxa- 5,9-undecadien-1-al), Hexahydro-4,7-methanoindan-1-carboxaldehyd, 2-Methyl octanal, alpha- Methyl-4-(I-methylethyl) benzeneacetaldehyd, 6,6-Dimethyl-2-norpinene-2-propionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9- Decenal, 3-Methyl-5-phenyl-1-pentanal, Methylnonyl acetaldehyd, 1-p-Menthene-q-carboxaldehyd, Citral, Lilial, 1-Decanal, Florhydral sowie 2,4-Dimethyl-3-cyclohexen-1-carboxaldehyd.

In einer bevorzugten Ausführungsform des Verfahrens dient es zur Freisetzung von Riechstoffketonen, insbesondere ausgewählt aus der Gruppe umfassend Buccoxim; Iso jasmon; Methyl beta naphthyl keton; Moschus indanon; Tonalid/Moschus plus; Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro- Beta-Ionon, Gamma-Methyl so genanntes Ionon, Fleuramon, Dihydrojasmon, Cis-Jasmon, Iso-E- Super, Methyl-cedrenyl-keton oder Methyl-cedrylon, Acetophenon, Methyl-acetophenon, Para- Methoxy-acetophenon, Methyl-beta-naphtyl-keton, Benzyl-aceton, Benzophenon, Para-Hydroxy- phenyl-butanon, Celery Keton oder Livescon, 6-Isopropyldecahydro-2-naphton, Dimethyl- octenon, Freskomenth, 4-(I-Ethoxy-vinyl)-3,3,5,5,-tetramethyl-cyclohexanon, Methyl-heptenon, 2-(2-(4-Methyl-3-cyclohexen-1-yl)-propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl- cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran sowie Hedion.

Ein weiterer Gegenstand der Erfindung ist ein Verfahren zur lang anhaltenden Beduftung von Oberflächen, wobei ein erfindungsgemäßes Wasch- oder Reinigungsmittel auf eine zu beduftende Oberfläche aufgebracht wird und die genannte Oberfläche anschließend einer elektromagnetischen Strahlung umfassend die Wellenlängen von 200 bis 400 nm ausgesetzt wird.

Ein weiterer Gegenstand der vorliegenden Erfindung liegt in der Verwendung von β-Hydroxyketonen der allgemeinen Formel (I) 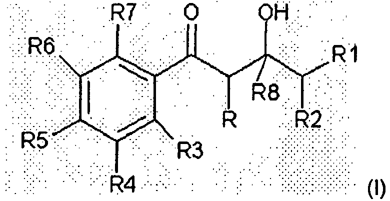 als Duftstoffvorläufer in Wasch- und Reinigungsmitteln, wobei die Reste R, R1, R2, R3, R4, R5, R6, R7, R8 definiert sind wie bereits angegeben.

Ebenso liegt ein weiterer Gegenstand der vorliegenden Erfindung in der Verwendung von β-Hydroxyketonen der allgemeinen Formel (I) 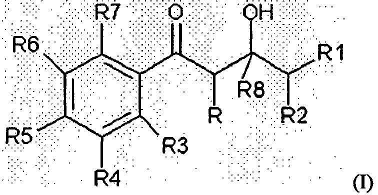 als Duftstoffvorläufer in kosmetischen Mitteln, wobei die Reste R, R1, R2, R3, R4, R5, R6, R7,R8 definiert sind wie bereits angegeben.

Ebenso liegt ein weiterer Gegenstand der vorliegenden Erfindung in der Verwendung von β-Hydroxyketonen der allgemeinen Formel (I) 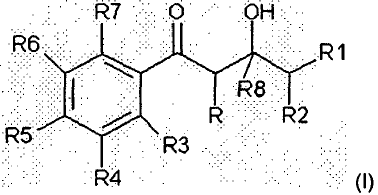 als Duftstoffvorläufer in Lufterfrischern, wobei die Reste R, R1, R2, R3, R4, R5, R6, R7, R8 definiert sind wie bereits angegeben.

Besonders vorteilhaft ist, dass die Duftstoffvorläufer bei Anwesenheit zusätzlicher Duftstoffe auch die Duftwirkung dieser Duftstoffe verlängern können. Ist die vorgenannte Verwendung also auf die Verlängerung der Duftwirkung von anderen Duftstoffen gerichtet, welche ebenfalls in dem Wasch- und Reinigungsmittel, kosmetischen Mittel oder Lufterfrischer enthalten sind, so liegt eine bevorzugte Ausführungsform der Erfindung vor. Ebenso liegt eine weitere bevorzugte Ausführungsform der Erfindung vor, wenn die vorgenannte Verwendung auf die Erzielung eines lange anhaltenden Frischegeruches gerichtet ist.

Wie bereits beschrieben wurde, sind Weichspüler bevorzugte Zusammensetzungen im Sinne der Erfindung. Ein erfindungsgemäßer Weichspüler kann üblicherweise eine weichmachende Komponente enthalten. Es ist ganz besonders bevorzugt, dass die weichmachende Komponente eine alkylierte, quaternäre Ammoniumverbindung ist, wobei mindestens eine Alkylkette durch eine Ester- oder Amidogruppe unterbrochen ist.

Die weichmachende Komponente umfasst beispielsweise quaternäre Ammoniumverbindungen wie Monoalk(en)yltrimethylammonium-Verbindungen, Dialk(en)yldimethylammonium-Verbindungen, Mono-, Di- oder Triester von Fettsäuren mit Alkanolaminen.

Geeignete Beispiele für quaternäre Ammoniumverbindungen sind beispielsweise in den Formeln (A-II) und (A-III) gezeigt: 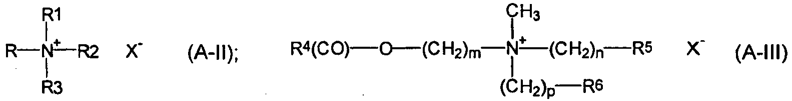 wobei in (A-II) R für einen acyclischen Alkylrest mit 12 bis 24 Kohlenstoffatomen, R¹ für einen gesättigten C₁-C₄ Alkyl- oder Hydroxyalkylrest steht, R² und R³ entweder gleich R oder R¹ sind oder für einen aromatischen Rest stehen. X- steht entweder für ein Halogenid-, Methosulfat-, Methophosphat- oder Phosphation sowie Mischungen aus diesen. Beispiele für kationische Verbindungen der Formel (II) sind Monotalgtrimethylammoniumchlorid, Monosteary-Itrimethylammoniumchlorid, Didecyldimethylammoniumchlorid, Ditalgdimethylammoniumchlorid oder Dihexadecylammoniumchlorid.

Verbindungen der Formel (A-III) und (A-IV) sind so genannte Esterquats. Esterquats zeichnen sich durch eine hervorragende biologische Abbaubarkeit aus. In Formel (A-III) steht R⁴ für einen aliphatischen Alk(en)ylrest mit 12 bis 22 Kohlenstoffatomen mit 0, 1, 2 oder 3 Doppelbindungen und/oder gegebenenfalls mit Substituenten; R⁵ steht für H, OH oder O(CO)R⁷, R⁶ steht unabhängig von R⁵ für H, OH oder O(CO)R⁸, wobei R⁷ und R⁸ unabhängig voneinander jeweils für einen aliphatischen Alk(en)ylrest mit 12 bis 22 Kohlenstoffatomen mit 0, 1, 2 oder 3 Doppelbindungen steht. m, n und p können jeweils unabhängig voneinander den Wert 1, 2 oder 3 haben. X⁻ kann entweder ein Halogenid-, Methosulfat-, Methophosphat- oder Phosphation sowie Mischungen aus diesen Anionen sein. Bevorzugt sind Verbindungen, bei denen R⁵ die Gruppe O(CO)R⁷ darstellt. Besonders bevorzugt sind Verbindungen, bei denen R⁵ die Gruppe O(CO)R⁷ darstellt und R⁴ und R⁷ Alk(en)ylreste mit 16, bis 18 Kohlenstoffatomen sind. Insbesondere bevorzugt sind Verbindungen, bei denen R⁶ zudem für OH steht. Beispiele für Verbindungen der Formel (A-II) sind Methyl-N-(2-hydroxyethyl)-N,N-di(talgacyloxyethyl)ammonium-methosulfat, Bis-(palmitoyloxyethyl)-hydroxyethyl-methyl-ammonium-methosulfat, 1,2-Bis-[talgacyloxy]-3-trimethylammoniumpro-panchlorid oder Methyl-N,N-bis(stearoyloxyethyl)-N-(2-hydroxyethyl)ammonium-methosulfat.

Werden quaternierte Verbindungen der Formel (A-III) eingesetzt, die ungesättigte Alkylketten aufweisen, sind die Acylgruppen bevorzugt, deren korrespondierenden Fettsäuren eine Jodzahl zwischen 1 und 100, bevorzugt zwischen 5 und 80, mehr bevorzugt zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere gleich oder größer als 60 : 40 haben. Handelsübliche Beispiele sind die von Stepan unter dem Warenzeichen Stepantex^{®} vertriebenen Methylhydroxyalkyldialkoyloxyalkylammoniummethosulfate oder die unter Dehyquart^{®} bekannten Produkte von Cognis, die unter Rewoquat^{®} bekannten Produkte von Degussa bzw. die unter Tetranyl® bekannten Produkte von Kao. Weitere bevorzugte Verbindungen sind die Diesterquats der Formel (A-IV), die unter dem Namen Rewoquat® W 222 LM bzw. CR 3099 erhältlich sind. 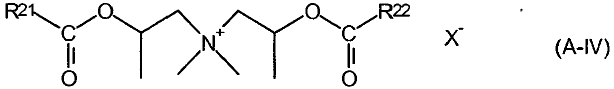 R²¹ und R²² stehen dabei unabhängig voneinander jeweils für einen aliphatischen Rest mit 12 bis 22 Kohlenstoffatomen mit 0, 1, 2 oder 3 Doppelbindungen.

Anstelle der Estergruppe O(CO)R²², wobei R²² für einen langkettigen Alk(en)ylrest steht, können weichmachende Verbindungen eingesetzt werden, die folgende Gruppen aufweisen: RO(CO), N(CO)R oder RN(CO) weisen, wobei von diesen Gruppen N(CO)R-Gruppen bevorzugt sind.

Weiterhin sind auch kationische Polymere geeignete weichmachende Komponente. Ebenfalls einsetzbar sind polyquaternierte Polymere (z.B. Luviquat® Care von BASF) und auch kationische Biopolymere auf Chitinbasis und deren Derivate, beispielsweise das unter der Handelsbezeichnung Chitosan® (Hersteller: Cognis) erhältliche Polymer.

Besonders bevorzugte weichmachende Komponenten sind alkylierte quaternäre Ammoniumverbindungen, von denen mindestens eine Alkylkette durch eine Estergruppe und/oder Amidogruppe unterbrochen ist. Ganz besonders bevorzugt sind N-Methyl-N-(2-hydroxyethyl)-N,N-(ditalgacyloxyethyl)ammonium-methosulfat oder Bis-(palmitoyloxyethyl)-hydroxyethyl-methylammonium-methosulfat.

Das erfindungsgemäße Wasch- oder Reinigungsmittel in Form von Weichspülern kann auch nichtionische weichmachende Komponenten enthalten, wie vor allem Polyoxyalkylenglycerolalkanoate, Polybutylene, langkettige Fettsäuren, ethoxylierte Fettsäureethanolamide, Alkylpolyglucoside, insbesondere Sorbitanmono,-di- und -triester, und Fettsäureester von Polycarbonsäuren.

In dem erfindungsgemäßen Weichspüler als erfindungsgemäßes Wasch- oder Reinigungsmittel ist die weichmachende Komponente in Mengen von z.B. 0,1 bis 80 Gew.-%, üblicherweise 1 bis 40 Gew.-%, vorzugsweise 2 bis 20 Gew.-%, insbesondere 3 bis 15 Gew.-% enthalten, und der zusätzliche Duftstoff vorteilhafterweise in Mengen von 0,1 bis 20 Gew.-%, vorzugsweise 1 bis 13 Gew.-%, insbesondere 2 bis 8 Gew.-%, jeweils bezogen auf die Gesamtmenge des erfindungsgemäßen Mittels, enthalten.

Als weitere Komponente kann ein erfindungsgemäßes Wasch- oder Reinigungsmittel, insbesondere aber ein Weichspüler, gegebenenfalls ein oder mehrere nichtionische Tenside enthalten, wobei solche eingesetzt werden können, die üblicherweise auch in Waschmitteln verwendet werden.

Als optionale nichtionische Tenside können vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt werden, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere optionale nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Die nichtionischen Tenside können in den erfindungsgemäßen Wasch- oder Reingungsmitteln vorzugsweise in Mengen von 0-30 Gew.-% enthalten sein, z.B. in Mengen > 0,1 Gew.-%. Es ist z.B. möglich, dass das Wasch- oder Reinigungsmittel 2 bis 30 Gew.-%, vorzugsweise 7 bis 20 Gew.-% und insbesondere 9 bis 15 Gew.-% Niotensid enthalten können, Gew.-% jeweils bezogen auf das gesamte Wasch- oder Reinigungsmittel.

Ein erfindungsgemäßes Wasch- oder Reinigungsmittel kann optional auch anionisches Tensid umfassen, z.B. in Mengen in Mengen von 0-30 Gew.-%, vorzugsweise > 0,1 Gew.-%. Es ist z.B. möglich, dass das Wasch- oder Reinigungsmittel 2 bis 30 Gew.-%, vorzugsweise 7 bis 20 Gew.-% und insbesondere 9 bis 15 Gew.-% Aniontensid enthalten können, Gew.-% jeweils bezogen auf das gesamte Wasch- oder Reinigungsmittel.

Als optionales anionisches Tensid kann beispielsweise solches vom Typ der Sulfonate und Sulfate eingesetzt werden. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium-oder Kaliumsalze, insbesondere in Form der Natriumsalze vor.

Es ist weiterhin bevorzugt, dass das erfindungsgemäße Wasch- oder Reinigungsmittel (z.B. Textil- oder Oberflächenbehandlungsmittel) zusätzlich weitere vorteilhafte Inhaltsstoffe enthält, die dem Fachmann grundsätzlich bekannt sind. So kann das erfindungsgemäße Wasch-oder Reinigungsmittel (z.B. Textil- oder Oberflächenbehandlungsmittel) weitere Inhaltsstoffe enthalten, die die anwendungstechnischen und/oder ästhetischen Eigenschaften des Mittels weiter verbessern. Im Rahmen der vorliegenden Erfindung enthalten bevorzugte Wasch- oder Reinigungsmittel zusätzlich einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Parfümträger, Fluoreszenzmittel, Farbstoffe, Hydrotope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber.

Als Gerüststoffe, die in den erfindungsgemäßen Wasch- oder Reinigungsmitteln optional enthalten sein können, z.B. in Mengen > 0,1 Gew.-% (bezogen auf das gesamte Wasch- oder Reinigungsmittel), sind beispielsweise Silikate, Aluminiumsilikate (insbesondere Zeolithe), Carbonate, Salze organischer Di- und Polycarbonsäuren sowie Mischungen dieser Stoffe zu nennen.

Ein erfindungsgemäßes Wasch- oder Reinigungsmittel kann optional ein Verdickungsmittel enthalten, z.B. in Mengen > 0,01 Gew.-% (bezogen auf das gesamte Wasch- oder Reinigungsmittel). Dies entspricht einer bevorzugten Ausführungsform der Erfindung. Das Verdickungsmittel kann beispielsweise einen Polyacrylat-Verdicker, Xanthan Gum, Gellan Gum, Guarkernmehl, Alginat, Carrageenan, Carboxymethylcellulose, Bentonite, Wellan Gum, Johannisbrotkernmehl, Agar-Agar, Tragant, Gummi arabicum, Pektine, Polyosen, Stärke, Dextrine, Gelatine und Casein umfassen.

Wasser kann in efindungsgemäßen flüssigen Wasch- oder Reinigungsmitteln enthalten sein, vorzugsweise in Mengen > 5 Gew.-%, z.B. In Mengen von 10-95 Gew.-%, vorzugsweise 20-80 Gew.-%, insbesondere 30-70 Gew.-%, Gew.-% bezogen auf das gesamte Wasch- oder Reinigungsmittel. Wasser kann auch in erfindungsgemäßen festen Wasch-oder Reinigungsmitteln enthalten sein, dann natürlich in entsprechend geringerer Menge, beispielsweise in Mengen < 5 Gew.-% oder z.B. < 3 Gew.-%.

Nichtwässrige Lösungsmittel, die in den erfindungsgemäßen (vorzugsweise flüssigen) Wasch- oder Reinigungsmiteln optional eingesetzt werden können, stammen beispielsweise aus der Gruppe der ein- oder mehrwertigen Alkohole, Alkanolamine oder Glykolether.

Die Viskosität der erfindungsgemäßen Wasch- oder Reinigungsmittel, so sie flüssig sind, kann mit üblichen Standardmethoden (beispielsweise Brookfield-Viskosimeter LVT-II bei 20 U/min und 20°C, Spindel 3) gemessen werden und beträgt vorzugsweise 20 bis 4000 mPas, wobei Werte zwischen 40 und 2000 mPas besonders bevorzugt sind. Insbesondere bevorzugt liegt die Viskosität von Weichspülern im Bereich von 40 bis 1000 mPas.

Um den pH-Wert der erfindungsgemäßen Wasch- oder Reinigungsmittel, so sie flüssig ist, in den gewünschten Bereich zu bringen, kann der Einsatz von pH-Stellmitteln angezeigt sein. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 7 Gew.-% oder vorzugsweise 5 Gew.-% der Gesamtformulierung nicht. Eine Untergrenze kann z.B. bei 0,1 Gew.-% liegen. Der pH-Wert der erfindungsgemäßen Wasch- oder Reinigungsmittel, so sie flüssig ist, liegt vorzugsweise zwischen 1 und 6 und bevorzugt zwischen 1,5 und 3,5.

Bei den erfindungsgemäßen Mitteln kann es sich um feste oder flüssige Formulierungen handeln, wobei feste Formulierungen als Pulver, Granulat, Extrudat, in Tab-Form, als Tablette oder als gepresster und/oder geschmolzener Formkörper vorliegen können. Bei flüssigen Formulierungen kann es sich um Lösungen, Emulsionen, Dispersionen Suspensionen, Mikroemulsionen, Gels oder Pasten handeln.

Die eingesetzten β-Hydroxyketone der allgemeinen Formel (I), 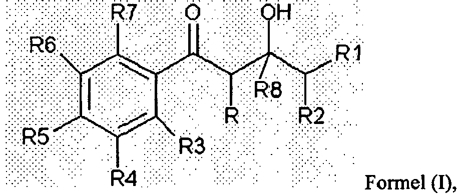 sind über eine Aldolreaktion von entsprechenden Verbindungen der Formel (IV), wie insbesondere Acetophenonderivaten oder Propiophenonderivaten, 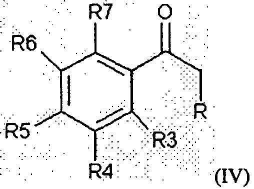 und Carbonylverbindungen der Formel (V) 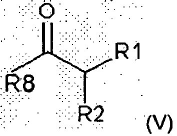 zugänglich, wobei die Reste R, R1, R2, R3, R4, R5, R6, R7, R8 definiert sind wie zuvor in Zusammenhang mit der Formel (I) bereits angegeben. Insbesondere sind sie über die Route der TiCl₄/NBu₃-induzierten Aldolreaktionen herstellbar (Literatur: Y. Tanabe et al., Tetrahedron 58 (2002), 8269-8280).

Die Aufreinigung der β-Hydroxyketone der allgemeinen Formel (I) erfolgt vorzugsweise durch Kristallisation, Destillation und/oder Säulenchromatographie.

### Beispiel

### a) Darstellung von (2R,3S)-3-hydroxy-2-methyl-1-phenyldecan-1-on

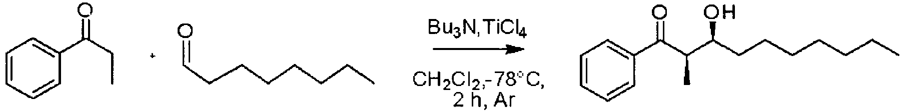

In einem ausgeheizten und mit Argon gefluteten Schlenk-Kolben wurden 2,7 ml (20 mmol) Propiophenon in 40 ml absoluten Dichlormethan vorgelegt. Diese Lösung wurde auf -78 °C gekühlt. Zu dieser Lösung wurden zuerst 24 ml einer 1 M-Titantetrachlorid-Lösung in Dichlormethan und anschließend 4,8 ml (28 mmol) Tributylamin mit einer Spritze dazugetropft. Nach 30 min Rühren bei -78 °C wurden 3,8 ml (24 mmol) Octanal dazu gegeben. Die Reaktionsmischung wurde weitere 2 Stunden bei -78 °C gerührt. Die Reaktionslösung wurde mit 40 ml Wasser gequencht und zwei Mal mit Diethylether extrahiert. Die organische Phase wurde mit Wasser, ges. NaCl-Lösung gewaschen und über MgSO₄ getrocknet. Das Filtrat wurde bei vermindertem Druck von Lösungsmittel befreit. Das Rohprodukt wurde säulenchromatographisch gereinigt (Petrolether:Ethylacetat=95:5).
Ausbeute: 80 %
Analytische Daten:
Rf=0.3
Sdp.= 175-180 °C
¹H NMR (300 MHz, CDCl₃): δ = 0.88 (3H, t, *J*=6.9 Hz), 1.25-1.58 (13H,m), 3.07 (s, 1H, O-H), 3.47 (1H, dq, *J*=2.8, 7.2 Hz), 4.03 (1H, m), 7.46-7.52 (2H,m), 7.53-7.62 (1H,m), 7.93-7.97 (2H,m).
¹³C NMR (300 MHz, CDCl₃): 11.02, 14.10, 22.65, 26.11, 29.26, 29.59, 31.82, 34.33 44.45, 71.34, 128.47, 128.77, 133.44, 135.92, 205.98.

### b) Belichtung von (2R,3S)-3-hydroxy-2-methyl-1-phenyldecan-1-on

(2R,3S)-3-hydroxy-2-methyl-1-phenyldecan-1-on wurde in Benzol gelöst. Die Reaktionslösung wurde in einem Multilamp-Photoreactor (8 W-Lampen (4×), Firma Luxchem) mit Emissionmaximum λ = 350 nm eine Stunde lang belichtet. Die Reaktion wurde mit Hilfe der GC/MS-Spektrometrie verfolgt. Nach spätestens 60 Minuten Belichtung wurde die weitgehend vollständige Umwandlung von (2R,3S)-3-hydroxy-2-methyl-1-phenyldecan-1-on in Phenylethylketon und Octanal beobachtet.

### c) Riechtest

Für den Riechtest wurden 0,2 mmol (2R,3S)-3-hydroxy-2-methyl-1-phenyldecan-1-on in 1 ml Dipropylenglykol gelöst. In die Lösung wurde ein Riechstreifen 2 cm tief eingetaucht, der anschließend unter Lichtausschluss bei 20°C getrocknet wurde. Auf diese Weise wurden 2 Riechstreifen hergestellt.

Nach erfolgreicher Trocknung wird der erste Riechstreifen über den gesamten Testzeitraum hinweg mit einer handelsüblichen Leuchtstoffröhre (gemäß DIN 5035 neutralweiß (nw); Farbtemperatur 3300 bis 5500 K) bestrahlt und die Duftintensität zu den jeweils angegebenen Zeitpunkten bestimmt.
Der zweite Riechstoffstreifen wurde in Dunkelheit gelagert.

Die Duftintensität wurde von 3 trainierten Probanden auf einer Skala von 0 bis 6 bewertet, wobei 6 die höchste Note ist und 0 für keine Duftwahrnehmung steht.

### Definition der Skalierung:

- 6: unangenehm stark
- 5: sehr stark
- 4: stark
- 3: intensiv
- 2: angenehm
- 1: wahrnehmbar
- 0: nicht mehr wahrnehmbar

Die Ergebnisse des Riechtests sind in der folgenden Tabelle dargestellt, wobei die angegebenen Werte den Bereich der Duftwahrnehmung der Probandengruppe widerspiegeln.

| | nach 30 Minuten | nach 60 Minuten | nach 90Minuten |
|---|---|---|---|
| Riechstoffstreifen 1 | 0-1 fettige Note | 2 fettige Note | 2 fettige Note |

Der Riechstoffstreifen 2, der in Dunkelheit gehandhabt wurde, weist weder nach 30, 60, 90 Minuten und auch nicht nach mehrstündiger Lagerung (z.B. 17 Stunden) einen Duft auf (entsprechend Wert 0 nach obiger Skala).
Es zeigte sich aber, dass der Riechstoffstreifen 2, nachdem er erst 17 Stunden in Dunkelheit war und danach, wie oben angegeben, bestrahlt wurde, bereits nach 60 Minuten einen angenehmen Dufteindruck hervorrief (entsprechend Wert 2 nach obiger Skala, fettige Note).

## Patentansprüche

1. Wasch- oder Reinigungsmittel enthaltend mindestens ein β-Hydroxyketon der allgemeinen Formel (I) 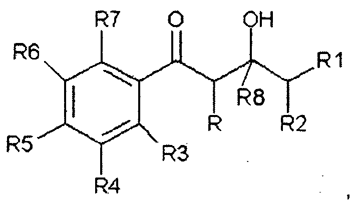 wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht,
und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono- oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen,
**dadurch gekennzeichnet, dass** das genannte β-Hydroxyketon der allgemeinen Formel (I) bei Einwirken elektromagnetischer Strahlung, insbesondere umfassend die Wellenlängen von 200 bis 400 nm, zumindest ein Riechstoffaldehyd oder ein Riechstoffketon freisetzt.

2. Wasch- oder Reinigungsmittel nach Anspruch 1, enthaltend mindestens ein β-Hydroxyketon der allgemeinen Formel (II) und/oder der allgemeinen Formel (III), 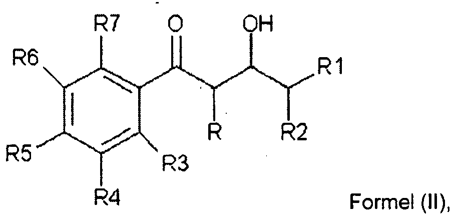 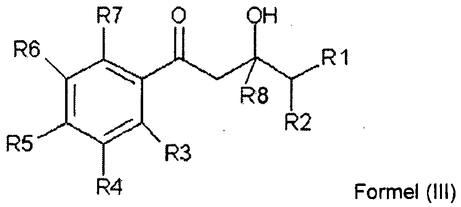 wobei in diesen Formeln die Reste R, R1, R2, R3, R4, R5, R6, R7, R8 definiert sind wie in Anspruch 1.

3. Wasch- oder Reinigungsmittel gemäß Anspruch 1 oder 2, **dadurch gekennzeichnet, dass** das β-Hydroxyketon bei Einwirken elektromagnetischer Strahlung umfassend die Wellenlängen von 200 bis 400 nm eine Verbindung der Formel (IV) 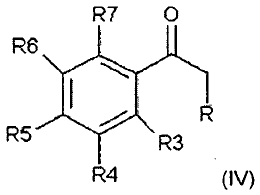 sowie eine Carbonylverbindung der Formel (V) freisetzt 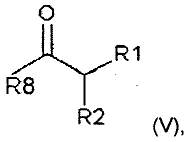 wobei die Reste R, R1, R2, R3, R4, R5, R6, R7, R8 definiert sind wie in Anspruch 1.

4. Wasch- oder Reinigungsmittel gemäß einem der Ansprüche 1 bis 3, **dadurch gekennzeichnet, dass** bei Einwirken elektromagnetischer Strahlung umfassend die Wellenlängen von 200 bis 400 nm zumindest ein Riechstoffaldehyd freigesetzt wird, insbesondere ausgewählt aus der Gruppe umfassend Melonal, Triplal, Ligustral, Adoxal; Anisaldehyd; Cymal; Ethylvanillin; Florhydral; Helional; Heliotropin; Hydroxycitronellal; Koavon; Laurinaldehyd; Lyral; Methyl-nonyl-acetaldehyd; p,t-Bucinal; Phenylacetaldehyd; Undecylenaldehyd; Vanillin;2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxy-phenylpropanal), 2-Methyl-4-(2,6,6-trimethyl-2(1)-cyclohexen-1-yl)bu-tanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acet-aldehyd, 4-Isopropylbenzyaldehyd, 1 ,2,3,4,5,6,7,8-octahydro-8,8-Dimethyl-2-naphthaldehyd, 2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2.1.0(2,6)]- decylidene-8)-butanal; Octahydro-J-methano-IH-indenecarboxaldehyd; 3-Ethoxy-4-hydroxybenzaldehyd, para-Ethyl-alpha, alpha-dimethylhydrozimt-aldehyd, alpha-Methyl-3,4- (methylenedioxy)-hydrozimtaldehyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyl octanal, Undecenal, 2,4,6-Trimethyl-3-cyclohexene-1-carboxaldehyd, 4-(3)(4-Methyl-3-pentenyl)-3- cyclohexen-carboxaldehyd, 1-Dodecanal, 2,4-Dimethyl cyclohexene-S-carboxaldehyd, 4-(4- Hydroxy-4-methyl pentyl-S-cylohexene-1-carboxaldehyd, 7-Methoxy-3,7-dimethyloctan-1-al, 2- Methyl undecanal, 2-Methyl decanal,1 -nonanal, 1-Octanal, 2,6,10-Trimethyl-5,9-undecadienal, 2- Methyl-3-(4-tertbutyl)propanal, Dihydrozimtaldehyd,1-methyl-4-(4-methyl-3-pentenyl)-3-cyclohexene-1-car-boxaldehyd, 5 oder 6 Methoxyhexahydro-4,7-methanoindan-1 oder 2- carboxyaldehyd; 3,7-Dimethyl-octan-1-al, 1 -Undecanal, 10-Undecen-1-al, 4-Hydroxy-3-methoxybenzaldehyd, 1 - Methyl-3-(4-methylpentyl)-3-cyclohexencarboxyaldehyd, 7-Hydroxy-3,7-dimethyl-octanal; trans-4-decenal, 2,6-Nonadienal, para-Tolylacetaldehyd; 4- Methylphenylacetaldehyd, 2-Methyl-4-(2,6,6-trimethyl-1-cyclo-hexen-1-yl)-2-butenal, ortho- Methoxyzimtaldehyd, S.S.δ-Trimethyl-S-cyclohexenecarboxaldehyd; 3,7-Dimethyl-2-methylene-6- octenal, Phenoxyacetaldehyd; 5,9-Dimethyl-4,8-decadienal, Peony aldehyd (6,10-dimethyl-3-oxa- 5,9-undecadien-1-al), Hexahydro-4,7-methanoindan-1-carboxaldehyd, 2-Methyl octanal, alpha- Methyl-4-(I-methylethyl) benzeneacetaldehyd, 6,6-Dimethyl-2-norpinene-2-propionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9- Decenal, 3-Methyl-5-phenyl-1-pentanal, Methylnonyl acetaldehyd, 1-p-Menthene-q-carboxaldehyd, Citral, Lilial, 1-Decanal, Florhydral sowie 2,4-Dimethyl-3-cyclohexen-1-carboxaldehyd.

5. Wasch- oder Reinigungsmittel gemäß einem der Ansprüche 1 bis 4, **dadurch gekennzeichnet, dass** bei Einwirken elektromagnetischer Strahlung umfassend die Wellenlängen von 200 bis 400 nm zumindest ein Riechstoffketon freigesetzt wird, insbesondere ausgewählt aus der Gruppe umfassend Buccoxim; Iso jasmon; Methyl beta naphthyl keton; Moschus indanon; Tonalid/Moschus plus; Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro- Beta-Ionon, Gamma-Methyl so genanntes Ionon, Fleuramon, Dihydrojasmon, Cis-Jasmon, Iso-E- Super, Methyl-cedrenyl-keton oder Methyl-cedrylon, Acetophenon, Methyl-acetophenon, Para- Methoxy-acetophenon, Methyl-beta-naphtyl-keton, Benzyl-aceton, Benzophenon, Para-Hydroxy- phenyl-butanon, Celery Keton oder Livescon, 6-Isopropyldecahydro-2-naphton, Dimethyloctenon, Freskomenth, 4-(I-Ethoxyvinyl)-3,3,5,5,-tetramethyl-cyclohexanon, Methyl-heptenon, 2-(2-(4- Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl- cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran sowie Hedion.

6. Wasch- oder Reinigungsmittel gemäß einem der Ansprüche 1 bis 5, **dadurch gekennzeichnet, dass** zumindest eines der folgenden β-Hydroxyketone gemäß den Formeln (VI)-(VII) enthalten ist:

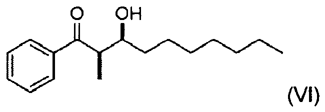

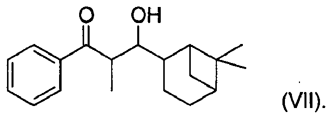

7. Wasch- oder Reinigungsmittel nach einem der Ansprüche 1 bis 6, **dadurch gekennzeichnet, dass** mindestens ein Tensid ausgewählt aus der Gruppe bestehend aus anionischen, kationischen, nichtionischen, zwitterionischen, amphoteren Tensiden oder Mischungen daraus enthalten ist bzw. sind.

8. Verfahren zur Freisetzungen von Carbonylverbindungen der Formel (V) 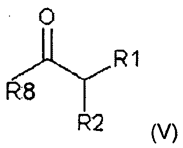 aus β-Hydroxyketonen der allgemeinen (Formel I) 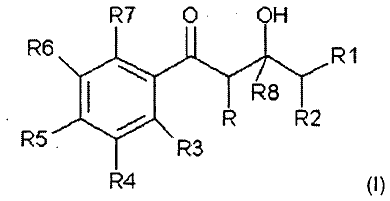 durch Einsatz elektromagnetischer Strahlung umfassend die Wellenlängen von 200 bis 400 nm, wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht,
und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono-oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

9. Verfahren gemäß Anspruch 8 zur Freisetzung von Riechstoffaldehyden, insbesondere ausgewählt aus der Gruppe umfassend Melonal, Triplal, Ligustral, Adoxal; Anisaldehyd; Cymal; Ethylvanillin; Florhydral; Helional; Heliotropin; Hydroxycitronellal; Koavon; Laurinaldehyd; Lyral; Methyl-nonyl-acetaldehyd; p,t-Bucinal; Phenylacetaldehyd; Undecylenaldehyd; Vanillin;2,6,10-Trimethyl-9-undecenal, 3-Dodecen-1-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1- yl)hutanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzyaldehyd, 1 ,2,3,4,5,6,7,8-octahydro-8,8- Dimethyl-2-naphthaldehyd, 2,4-Dimethyl-3-cyclohexen-1-carboxy-aldehyd, 2-Methyl-3- (isopropylphenyl)propanal, Decyl aldehyd, 2,6-Dimethyl-5-heptenal; 4-(tricyclo[5.2. 1.0 (2,6)]- decylidene-8)-butanal; Octahydro-J-methano-IH-indenecarboxaldehyd; 3-Ethoxy-4- hydroxybenzaldehyd, para-Ethyl-alpha, alpha-dimethylhydrozimtaldehyd, alpha-Methyl-3,4- (methylenedioxy)-hydrozimtaldehyd, 3,4-Methylenedioxybenzaldehyd, alpha-n-Hexylzimt-aldehyd, m-Cymene-7-carboxaldehyd, alpha-Methylphenylacetaldehyd, 7-Hydroxy-3,7-dimethyl octanal, Undecenal, 2,4,6-Trimethyl-3-cyclohexene-1-carboxaldehyd, 4-(3)(4-Methyl-3-pentenyl)-3-cyclohexen-carboxaldehyd, 1-Dodecanal, 2,4-Dimethyl cyclohexene-S-carboxaldehyd, 4-(4-Hydroxy-4-methyl pentyl-S-cylohexene-1-carboxaldehyd, 7-Methoxy-3,7-dimethyloctan-1-al, 2- Methyl undecanal, 2-Methyl decanal,1 -nonanal, 1-Octanal, 2,6,10-Trimethyl-5,9-undecadienalₗ 2- Methyl-3-(4-tertbutyl)propanal, Dihydrozimtaldehyd,1-methyl-4-(4-methyl-3-pentenyl)-3- cyclohexene-1-carboxaldehyd, 5 oder 6 Methoxyhexahydro-4,7-methanoindan-1 oder 2- carboxyaldehyd; 3,7-Dimethyloctan-1-al, 1 -Undecanal, 10-Undecen-1-al, 4-Hydroxy-3- methoxybenzaldehyd, 1 -Methyl-3-(4-methylpentyl)-3-cyclohexencarboxyaldehyd, 7-Hydroxy-3,7- dimethyl-octanal; trans-4-decenal, 2,6-Nonadienal, para-Tolylacetaldehyd; 4- Methylphenylacetaldehyd, 2-Methyl-4-(2,6,6-trimethyl-1-cyclohexen-1-yl)-2-butenal, ortho- Methoxyzimtaldehyd, S.S.δ-Trimethyl-S-cyclohexencarbox-aldehyd; 3,7-Dimethyl-2-methylene-6- octenal, Phenoxyacetaldehyd; 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha- Methyl-4-(I-methylethyl) benzeneacetaldehyd, 6,6-Dimethyl-2-norpinene-2-propionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9- Decenal, 3-Methyl-5-phenyl-1-pentanal, Methylnonyl acetaldehyd, 1-p-Menthene-q-carboxaldehyd, Citral, Lilial, 1-Decanal, Florhydral sowie 2,4-Dimethyl-3-cyclohexen-1- carboxaldehyd.

10. Verfahren gemäß Anspruch 8 zur Freisetzung von Riechstoffketonen, insbesondere ausgewählt aus der Gruppe umfassend Buccoxim; Iso jasmon; Methyl beta naphthyl keton; Moschus indanon; Tonalid/Moschus plus; Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro- Beta-Ionon, Gamma-Methyl so genanntes Ionon, Fleuramon, Dihydrojasmon, Cis-Jasmon, Iso-E- Super, Methyl-cedrenyl-keton oder Methyl-cedrylon, Acetophenon, Methyl-aceto-phenon, Para- Methoxy-acetophenon, Methyl-beta-naphtyl-keton, Benzylaceton, Benzophenon, Para-Hydroxy- phenyl-butanon, Celery Keton oder Livescon, 6-Isopropyldecahydro-2-naphton, Dimethyl- octenon, Freskomenth, 4-(1-Ethoxyvinyl)-3,3,5,5,-tetramethylcyclohexanon, Methyl-heptenon, 2-(2-(4- Methyl-3-cyclohexen-1-yl)propyl)-cyclopentanon, 1-(p-Menthen-6(2)-yl)-1-propanon, 4-(4-Hydroxy- 3-methoxyphenyl)-2-butanon, 2-Acetyl-3,3-dimethyl-norbornan, 6,7-Dihydro-1 ,1 ,2,3,3-pentamethyl- 4(5H)-indanon, 4-Damascol, Dulcinyl or Cassion, Gelson, Hexalon, Isocyclemon E, Methyl- cyclocitron, Methyl-Lavendel-keton, Orivon, Para-tert-butyl-cyclohexanon, Verdon, Delphon, Muscon, Neobutenon, Plicaton, Velouton, 2,4,4,7-Tetra-methyl-oct-6-en-3-on, Tetrameran sowie Hedion.

11. Verfahren zur lang anhaltenden Beduftung von Oberflächen, **dadurch gekennzeichnet, dass** eine Zusammensetzung nach einem der Ansprüche 1 bis 7 auf eine zu beduftende Oberfläche aufgebracht wird und die genannte Oberfläche anschließend einer elektromagnetischen Strahlung umfassend die Wellenlängen von 200 bis 400 nm ausgesetzt wird.

12. Verwendung von β-Hydroxyketonen der allgemeinen Formel (I) 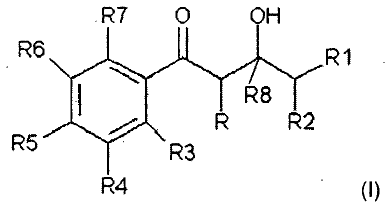 als Duftstoffvorläufer in Wasch- und Reinigungsmitteln, wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht,
und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono-oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

13. Verwendung von β-Hydroxyketonen der allgemeinen Formel (I) 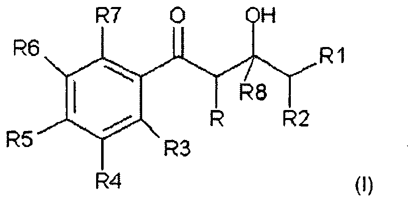 als Duftstoffvorläufer in kosmetischen Mitteln,
wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht,
und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono-oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

14. Verwendung von β-Hydroxyketonen der allgemeinen Formel (I) 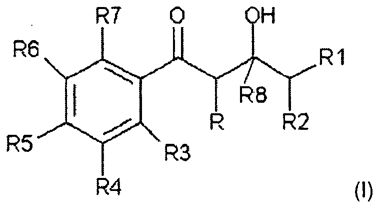 als Duftstoffvorläufer in Lufterfrischern,
wobei in dieser Formel, unabhängig voneinander, R und R8 für ein Wasserstoffatom oder für einen organischen Rest, insbesondere für eine lineare oder verzweigte, substituierte oder unsubstituierte Alkyl- oder Alkylengruppe von C₁ bis C₂₀ steht, vorzugsweise aber für einen Methylrest steht,
und wobei R1 und R2, unabhängig voneinander für ein Wasserstoffatom oder für organische Reste, wie insbesondere eine lineare oder verzweigte, substituierte oder unsubstituierte Alkyl- oder Alkylengruppe von C₁ bis C₂₀, vorzugsweise C₄ bis C₂₀, stehen, wobei die Reste R1 und R2 zusammen auch ein Ringsystem bilden können, insbesondere eine unsubstituierte oder substituierte Mono-oder Polycycloalkylgruppe von C₃ bis C₈ oder eine unsubstituierte oder substituierte Phenylgruppe darstellen können,
und wobei R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

## Claims

1. A washing or cleaning agent containing at least one β-hydroxy ketone of the general formula (I) 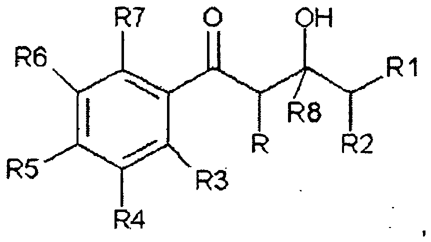 wherein in this formula, mutually independently, R and R8 denote a hydrogen atom or an organic residue, in particular a linear or branched, substituted or unsubstituted alkyl or alkylene group from C₁ to C₂₀, but preferably a methyl residue,
and wherein R1 and R2 mutually independently denote a hydrogen atom or organic residues, such as in particular a linear or branched, substituted or unsubstituted alkyl or alkylene group from C₁ to C₂₀, preferably C₄ to C₂₀, wherein the R1 and R2 residues together can also represent a ring system, in particular an unsubstituted or substituted mono- or polycycloalkyl group from C₃ to C₈ or an unsubstituted or substituted phenyl group,
and wherein R3, R4, R5, R6 and R7 mutually independently denote hydrogen, a halogen atom, NO₂, a linear or branched, substituted or unsubstituted alkoxy group having 1 to 15 carbon atoms or a linear or branched, substituted or unsubstituted alkyl group having 1 to 15 carbon atoms,
**characterized in that** upon the action of electromagnetic radiation, in particular encompassing the wavelengths from 200 to 400 nm, the aforesaid β-hydroxy ketone of the general formula (I) releases at least one fragrance aldehyde or one fragrance ketone.

2. The washing or cleaning agent according to Claim 1, containing at least one β-hydroxy ketone of the general formula (II) and/or the general formula (III) 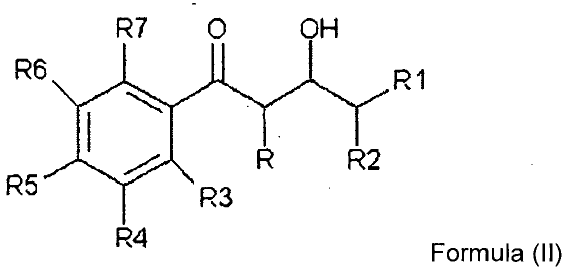 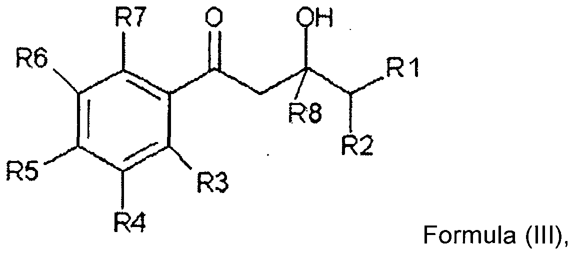 wherein in these formulas the residues R, R1, R2, R3, R4, R5, R6, R7, R8 are defined as in Claim 1.

3. The washing or cleaning agent according to Claim 1 or 2, **characterized in that** upon the action of electromagnetic radiation comprising the wavelengths from 200 to 400 nm the β-hydroxy ketone releases a compound of formula (IV) 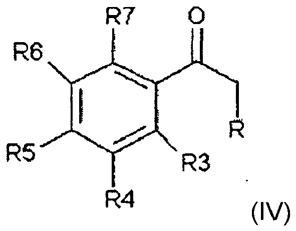 as well as a carbonyl compound of formula (V) 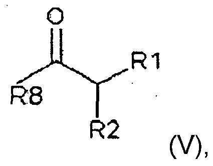 wherein the residues R, R1, R2, R3, R4, R5, R6, R7, R8 are defined as in Claim 1.

4. The washing or cleaning agent according to one of Claims 1 to 3, **characterized in that** upon the action of electromagnetic radiation comprising the wavelengths from 200 to 400 nm at least one fragrance aldehyde is released, selected in particular from the group comprising melonal, triplal, ligustral, adoxal; anisaldehyde; cymal; ethyl vanillin; Florhydral; helional; heliotropin; hydroxycitronellal; Koavone; lauryl aldehyde; lyral; methyl nonyl acetaldehyde; p,t-bucinal; phenyl acetaldehyde; undecylene aldehyde; vanillin; 2,6,10-trimethyl-9-undecenal, 3-dodecen-1-al, α-n-amylcinnamaldehyde, 4-methoxybenzaldehyde, benzaldehyde, 3-(4-tert-butylphenyl)propanal, 2-methyl-3-(para-methoxyphenylpropanal), 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzyl aldehyde, 1,2,3,4,5,6,7,8-octahydro-8,8-dimethyl-2-naphthaldehyde, 2,4-dimethyl-3-cyclohexene-1-carboxyaldehyde, 2-methyl-3-(isopropylphenyl)propanal, decyl aldehyde, 2,6-dimethyl-5-heptenal, 4-(tricyclo[5.2.1.0(2,6)]-decylidene-8) butanal; octahydro-J-methano-IH-indene carboxaldehyde, 3-ethoxy-4-hydroxybenzaldehyde, para-ethyl-α,α-dimethylhydrocinnamaldehyde, α-methyl-3,4-(methylenedioxy)hydrocinnamaldehyde, 3,4-methylenedioxybenzaldehyde, α-n-hexylcinnamaldehyde, m-cymene-7-carboxaldehyde, α-methyl phenyl acetaldehyde, 7-hydroxy-3,7-dimethyloctanal; undecenal, 2,4,6-trimethyl-3-cyclohexene-1-carboxaldehyde, 4-(3)(4-methyl-3-pentenyl)-3-cyclohexene carboxaldehyde, 1-dodecanal, 2,4-dimethylcyclohexene-S-carboxaldehyde, 4-(4-hydroxy-4-methylpentyl-S-cyclohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanoindane-1- or -2-carboxyaldehyde; 3,7-dimethyloctan-1-al, 1-undecanal, 10-undecen-1-al, 4-hydroxy-3-methoxybenzaldehyde, 1-methyl-3-(4-methylpentyl)-3-cyclohexene carboxyaldehyde, 7-hydroxy-3,7-dimethyloctanal; trans-4-decenal, 2,6-nonadienal, para-tolylacetaldehyde; 4-methyl phenyl acetaldehyde, 2-methyl-4-(2,6,6-trimethyl-1-cyclohexen-1-yl)-2-butenal, ortho-methoxycinnamaldehyde, S,S,δ-trimethyl-S-cyclohexene carboxaldehyde; 3,7-dimethyl-2-methylene-6-octenal, phenoxyacetaldehyde; 5,9-dimethyl-4,8-decadienal, peony aldehyde (6,10-dimethyl-3-oxa-5,9-undecadien-1-al), hexahydro-4,7-methanoindane-1-carboxaldehyde, 2-methyloctanal, α-methyl-4-(I-methylethyl)benzeneacetaldehyde, 6,6-dimethyl-2-norpinene-2-propionaldehyde, para-methylphenoxyacetaldehyde; 2-methyl-3-phenyl-2-propen-1-al, 3,5,5-trimethylhexanal, hexahydro-8,8-dimethyl-2-naphthaldehyde, 3-propylbicyclo[2.2.1]hept-5-ene-2-carbaldehyde, 9-decenal, 3-methyl-5-phenyl-1-pentanal, methyl nonyl acetaldehyde, 1-p-menthene-q-carboxaldehyde, citral, lilial, 1-decanal, Florhydral and 2,4-dimethyl-3-cyclohexene-1-carboxaldehyde.

5. The washing or cleaning agent according to one of Claims 1 to 4, **characterized in that** upon the action of electromagnetic radiation comprising the wavelengths from 200 to 400 nm at least one fragrance ketone is released, in particular selected from the group comprising buccoxime; isojasmone; methyl β-naphthyl ketone; musk indanone; tonalide (Musk Plus); α-damascone, β-damascone, δ-damascone, isodamascone, damascenone, damarose, methyl dihydrojasmonate, menthone, carvone, camphor, fenchone, α-ionene, β-ionone, dihydro-β-ionone, γ-methyl so-called ionone, fleuramone, dihydrojasmone, cis-jasmone, iso-E-super, methyl cedrenyl ketone or methylcedrylone, acetophenone, methylacetophenone, para-methoxyacetophenone, methyl β-naphtyl ketone, benzyl acetone, benzophenone, para-hydroxyphenylbutanone, celery ketone or livescone, 6-isopropyldecahydro-2-naphthone, dimethyloctenone, Freskomenth, 4-(I-ethoxyvinyl)-3,3,5,5-tetramethylcyclohexanone, methylheptenone, 2-(2-(4-methyl-3-cyclohexene-1-yl)propyl)-cyclopentanone, 1-(p-menthen-6(2)-yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)-indanone, 4-damascol, dulcinyl or cassion, gelsone, hexalon, isocyclemone E, methyl cyclocitrone, methyl lavendel ketone, orivone, para-tert-butylcyclohexanone, verdone, delphone, muscone, neobutenone, plicatone, veloutone, 2,4,4,7-tetramethyloct-6-en-3-one, tetrameran and hedione.

6. The washing or cleaning agent according to one of Claims 1 to 5, **characterized in that** it contains at least one of the following β-hydroxy ketones according to formulas (VI) to (VII):

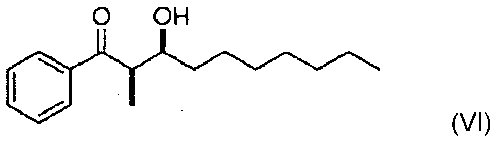

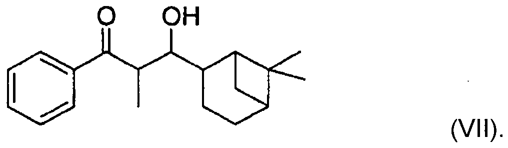

7. The washing or cleaning agent according to one of Claims 1 to 6, **characterized in that** it contains at least one surfactant selected from the group consisting of anionic, cationic, nonionic, zwitterionic, amphoteric surfactants or mixtures thereof.

8. A method for release of carbonyl compounds of formula (V) 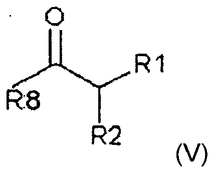 from β-hydroxy ketones of the general formula (I) 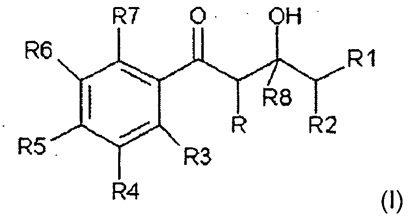 by the use of electromagnetic radiation comprising the wavelengths of 200 to 400 nm,
wherein in this formula, mutually independently, R and R8 denote a hydrogen atom or an organic residue, in particular a linear or branched, substituted or unsubstituted alkyl or alkylene group from C₁ to C₂₀, but preferably a methyl residue,
and wherein R1 and R2 mutually independently denote a hydrogen atom or organic residues, such as in particular a linear or branched, substituted or unsubstituted alkyl or alkylene group from C₁ to C₂₀, preferably C₄ to C₂₀, wherein the R1 and R2 residues together can also represent a ring system, in particular an unsubstituted or substituted mono- or polycycloalkyl group from C₃ to C₈ or an unsubstituted or substituted phenyl group,
and wherein R3, R4, R5, R6 and R7 mutually independently denote hydrogen, a halogen atom, NO₂, a linear or branched, substituted or unsubstituted alkoxy group having 1 to 15 carbon atoms or a linear or branched, substituted or unsubstituted alkyl group having 1 to 15 carbon atoms.

9. The method according to Claim 8 for the release of odorant aldehydes, selected in particular from the group comprising melonal, triplal, ligustral, adoxal; anisaldehyde; cymal; ethyl vanillin; Florhydral; helional; heliotropin; hydroxycitronellal; Koavone; lauryl aldehyde; lyral; methyl nonyl acetaldehyde; p,t-bucinal; phenyl acetaldehyde; undecylene aldehyde; vanillin; 2,6,10-trimethyl-9-undecenal, 3-dodecen-1-al, α-n-amylcinnamaldehyde, 4-methoxybenzaldehyde, benzaldehyde, 3-(4-tert-butylphenyl)propanal, 2-methyl-3-(para-methoxyphenylpropanal), 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzyl aldehyde, 1,2,3,4,5,6,7,8-octahydro-8,8-dimethyl-2-naphthaldehyde, 2,4-dimethyl-3-cyclohexene-1-carboxyaldehyde, 2-methyl-3-(isopropylphenyl)propanal, decyl aldehyde, 2,6-dimethyl-5-heptenal, 4-(tricyclo[5.2.1.0(2,6)]-decylidene-8) butanal; octahydro-J-methano-IH-indene carboxaldehyde, 3-ethoxy-4-hydroxybenzaldehyde, para-ethyl-α,α-dimethylhydrocinnamaldehyde, α-methyl-3,4-(methylenedioxy)hydrocinnamaldehyde, 3,4-methylenedioxybenzaldehyde, α-n-hexylcinnamaldehyde, m-cymene-7-carboxaldehyde, α-methyl phenyl acetaldehyde, 7-hydroxy-3,7-dimethyloctanal; undecenal, 2,4,6-trimethyl-3-cyclohexene-1-carboxaldehyde, 4-(3)(4-methyl-3-pentenyl)-3-cyclohexene carboxaldehyde, 1-dodecanal, 2,4-dimethylcyclohexene-S-carboxaldehyde, 4-(4-hydroxy-4-methylpentyl-S-cyclohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanoindane-1- or 2-carboxyaldehyde; 3,7-dimethyloctan-1-al, 1-undecanal, 10-undecen-1-al, 4-hydroxy-3-methoxybenzaldehyde, 1-methyl-3-(4-methylpentyl)-3-cyclohexene carboxyaldehyde, 7-hydroxy-3,7-dimethyloctanal; trans-4-decenal, 2,6-nonadienal, para-tolylacetaldehyde; 4-methyl phenyl acetaldehyde, 2-methyl-4-(2,6,6-trimethyl-1-cyclohexen-1-yl)-2-butenal, ortho-methoxycinnamaldehyde, S,S,δ-trimethyl-S-cyclohexene carboxaldehyde; 3,7-dimethyl-2-methylene-6-octenal, phenoxyacetaldehyde; 5,9-dimethyl-4,8-decadienal, peony aldehyde (6,10-dimethyl-3-oxa-5,9-undecadien-1-al), hexahydro-4,7-methanoindane-1-carboxaldehyde, 2-methyloctanal, α-methyl-4-(1-methylethyl)benzeneacetaldehyde, 6,6-dimethyl-2-norpinene-2-propionaldehyde, para-methylphenoxyacetaldehyde; 2-methyl-3-phenyl-2-propen-1-al, 3,5,5-trimethylhexanal, hexahydro-8,8-dimethyl-2-naphthaldehyde, 3-propylbicyclo[2.2.1]hept-5-ene-2-carbaldehyde, 9-decenal, 3-methyl-5-phenyl-1-pentanal, methyl nonyl acetaldehyde, 1-p-menthene-q-carboxaldehyde, citral, lilial, 1-decanal, Florhydral and 2,4-dimethyl-3-cyclohexene-1-carboxaldehyde.

10. The method according to Claim 8 for the release of fragrance ketones, selected in particular from the group comprising buccoxime; isojasmone; methyl β-naphthyl ketone; musk indanone; tonalide (Musk Plus); α-damascone, β-damascone, δ-damascone, isodamascone, damascenone, damarose, methyl dihydrojasmonate, menthone, carvone, camphor, fenchone, α-ionene, β-ionone, dihydro-β-ionone, γ-methyl so-called ionone, fleuramone, dihydrojasmone, cis-jasmone, iso-E-super, methyl cedrenyl ketone or methylcedrylone, acetophenone, methylacetophenone, para-methoxyacetophenone, methyl β-naphtyl ketone, benzyl acetone, benzophenone, para-hydroxyphenylbutanone, celery ketone or livescone, 6-isopropyldecahydro-2-naphthone, dimethyloctenone, Freskomenth, 4-(I-ethoxyvinyl)-3,3,5,5-tetramethylcyclohexanone, methylheptenone, 2-(2-(4-methyl-3-cyclohexene-1-yl)propyl)-cyclopentanone, 1-(p-menthen-6(2)-yl)-1-propanone, 4-(4-hydroxy-3-methoxyphenyl)-2-butanone, 2-acetyl-3,3-dimethylnorbornane, 6,7-dihydro-1, 1,2,3,3-pentamethyl-4(5H)-indanone, 4-damascol, dulcinyl or cassion, gelsone, hexalon, isocyclemone E, methyl cyclocitrone, methyl lavendel ketone, orivone, para-tert-butylcyclohexanone, verdone, delphone, muscone, neobutenone, plicatone, veloutone, 2,4,4,7-tetramethyloct-6-en-3-one, tetrameran and hedione.

11. A method for long-lasting scenting of surfaces, **characterized in that** a composition according to one of Claims 1 to 7 is applied onto a surface to be scented and said surface is then exposed to an electromagnetic radiation comprising the wavelengths from 200 to 400 nm.

12. Use of β-hydroxy ketones of the general formula (I) 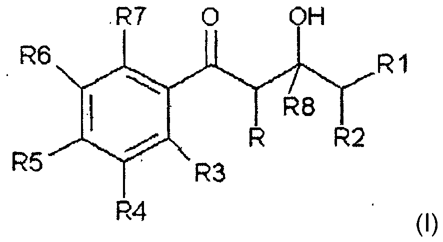 as scent precursors in washing and cleaning agents, wherein in this formula, mutually independently, R and R8 denote a hydrogen atom or an organic residue, in particular a linear or branched, substituted or unsubstituted alkyl or alkylene group from C₁ to C₂₀, but preferably a methyl residue,
and wherein R1 and R2 mutually independently denote a hydrogen atom or organic residues, such as in particular a linear or branched, substituted or unsubstituted alkyl or alkylene group from C₁ to C₂₀, preferably C₄ to C₂₀, wherein the R1 and R2 residues together can also represent a ring system, in particular an unsubstituted or substituted mono- or polycycloalkyl group from C₃ to C₈ or an unsubstituted or substituted phenyl group,
and wherein R3, R4, R5, R6 and R7 mutually independently denote hydrogen, a halogen atom, NO₂, a linear or branched, substituted or unsubstituted alkoxy group having 1 to 15 carbon atoms or a linear or branched, substituted or unsubstituted alkyl group having 1 to 15 carbon atoms.

13. Use of β-hydroxy ketones of the general formula (I) 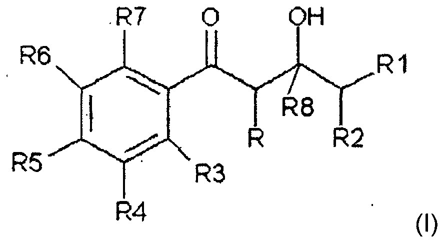 as scent precursors in cosmetic agents,
wherein in this formula, mutually independently, R and R8 denote a hydrogen atom or an organic residue, in particular a linear or branched, substituted or unsubstituted alkyl or alkylene group from C₁ to C₂₀, but preferably a methyl residue,
and wherein R1 and R2 mutually independently denote a hydrogen atom or organic residues, such as in particular a linear or branched, substituted or unsubstituted alkyl or alkylene group from C₁ to C₂₀, preferably C₄ to C₂₀, wherein the R1 and R2 residues together can also represent a ring system, in particular an unsubstituted or substituted mono- or polycycloalkyl group from C₃ to C₈ or an unsubstituted or substituted phenyl group,
and wherein R3, R4, R5, R6 and R7 mutually independently denote hydrogen, a halogen atom, NO₂, a linear or branched, substituted or unsubstituted alkoxy group having 1 to 15 carbon atoms or a linear or branched, substituted or unsubstituted alkyl group having 1 to 15 carbon atoms.

14. Use of β-hydroxy ketones of the general formula (I) 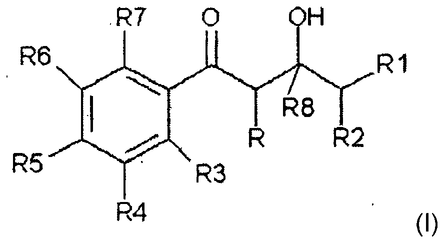 as scent precursors in air fresheners, wherein in this formula, mutually independently, R and R8 denote a hydrogen atom or an organic residue, in particular a linear or branched, substituted or unsubstituted alkyl or alkylene group from C₁ to C₂₀, but preferably a methyl residue,
and wherein R1 and R2 mutually independently denote a hydrogen atom or organic residues, such as in particular a linear or branched, substituted or unsubstituted alkyl or alkylene group from C₁ to C₂₀, preferably C₄ to C₂₀, wherein the R1 and R2 residues together can also represent a ring system, in particular an unsubstituted or substituted mono- or polycycloalkyl group from C₃ to C₈ or an unsubstituted or substituted phenyl group,
and wherein R3, R4, R5, R6 and R7 mutually independently denote hydrogen, a halogen atom, NO₂, a linear or branched, substituted or unsubstituted alkoxy group having 1 to 15 carbon atoms or a linear or branched, substituted or unsubstituted alkyl group having 1 to 15 carbon atoms.

## Revendications

1. Agent de lavage ou de nettoyage contenant au moins une β-hydroxycétone répondant à la formule générale (I) 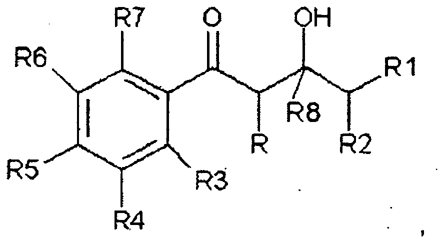 dans lequel, dans cette formule, R et R8 représentent, indépendamment l'un de l'autre, un atome d'hydrogène ou un résidu organique, en particulier un groupe alkyle ou alkylène linéaire ou ramifié, substitué ou non substitué contenant de 1 à 20 atomes de carbone, mais de préférence un résidu méthyle,
et dans lequel R1 et R2, représentent, indépendamment l'un de l'autre, un atome d'hydrogène ou des résidus organiques par exemple en particulier un groupe alkyle ou un groupe alkylène linéaire ou ramifié, substitué ou non substitué, contenant de 1 à 20 atomes de carbone, de préférence de 4 à 20 atomes de carbone, les résidus R1 et R2 pouvant également former ensemble un système nucléaire, en particulier un groupe monocycloalkyle ou un groupe polycycloalkyle non substitué ou substitué contenant de 3 à 8 atomes de carbone ou un groupe phényle non substitué ou substitué,
et dans lequel R3, R4, R5, R6 et R7 représentent, indépendamment l'un de l'autre, un atome d'hydrogène, un atome d'halogène, un groupe NO₂, un groupe alcoxy linéaire ou ramifié, substitué ou non substitué contenant de 1 à 15 atomes de carbone ou un groupe alkyle linéaire ou ramifié, substitué ou non substitué contenant de 1 à 15 atomes de carbone,
**caractérisé en ce que** la β-hydroxycétone mentionnée répondant à la formule générale (I) libère, par exposition à un rayonnement électromagnétique comprenant en particulier les longueurs d'ondes de 200 à 400 nm, au moins un aldéhyde faisant office de parfum ou une cétone faisant office de parfum.

2. Agent de lavage ou de nettoyage selon la revendication 1, contenant au moins une β-hydroxycétone répondant à la formule générale (II) et/ou à la formule générale (III), 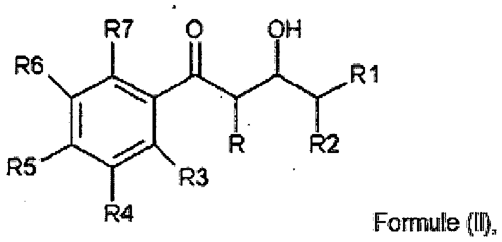 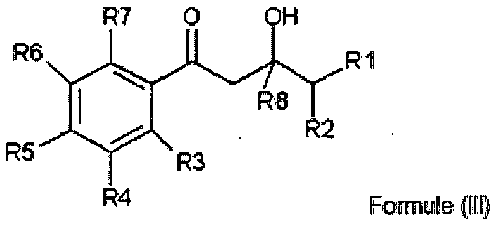 dans lequel, dans ces formules, les résidus R, R1, R2, R3, R4, R5, R6, R7, R8 sont tels que définis à la revendication 1.

3. Agent de lavage ou de nettoyage selon la revendication 1 ou 2, **caractérisé en ce que** la β-hydroxycétone libère, par exposition à un rayonnement électromagnétique comprenant les longueurs d'onde de 200 à 400 nm, un composé répondant à la formule (IV) 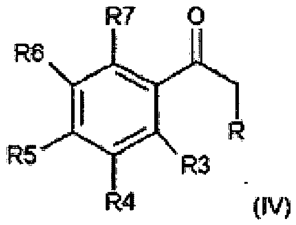 ainsi qu'un composé carbonyle répondant à la formule (V) 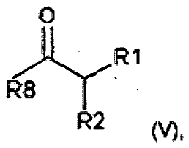 dans lequel les résidus R, R1, R2, R3, R4, R5, R6, R7, R8 sont tels que définis à la revendication 1.

4. Agent de lavage ou de nettoyage selon l'une quelconque des revendications 1 à 3, **caractérisé en ce que**, par exposition à un rayonnement électromagnétique comprenant les longueurs d'onde de 200 à 400 nm, est libéré au moins un aldéhyde faisant office de parfum, choisi en particulier parmi le groupe comprenant le mélonal, le triplal, le ligustral, l'adoxal; l'anisaldéhyde; le cymal; l'éthylvanilline ; le florhydral ; l'hélional ; l'héliotropine ; l'hydroxycitronellal ; la koavone ; le laurinaldéhyde ; le lyral ; le méthyl-nonyl-acétaldéhyde ; le p,t-bucinal ; le phénylacétaldéhyde ; le undécylène-aldéhyde ; la vanilline ; le 2,6,10-triméthyl-9-undécénal, le 3-dodécén-1-al, l'alpha-n-amyl-cinnamaldéhyde, le 4-méthoxybenzaldéhyde, le benzaldéhyde, le 3-(4-tert-butylphényl)-propanal, le 2-méthyl-3-(para-méthoxy-phénylpropanal), le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzyaldéhyde, le 1,2,3,4,5,6,7,8-octahydro-8,8-diméthyl-2-naphtaldéhyde, le 2,4-diméthyl-3-cyclohexén-1-carboxyaldéhyde, le 2-méthyl-3-(isopropylphényl)propanal, le décylaldéhyde, le 2,6-diméthyl-5-hepténal ; le 4-(tricyclo[5.2.1.0(2,6)]-décylidène-8)-butanal ; l'octahydro-J-méthano-IH-indènecarboxaldéhyde ; le 3-éthoxy-4-hydroxybenzaldéhyde, le para-éthyl-alpha,alpha-diméthylhydro-cinnamaldéhyde, l'alpha-méthyl-3,4-(méthylènedioxy)-hydrocinnamaldéhyde, le 3,4-méthylènedioxybenzaldéhyde, l'alpha-n-hexylcinnamaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexène-carboxaldéhyde, le 1-dodécanal, le 2,4-diméthylcyclohexène-S-carboxaldéhyde, le 4-(4-hydroxy-4-méthylpentyl-S-cylohexène-1-carboxaldéhyde, le 7-méthoxy-3,7-diméthyl-octan-1-al, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5 ou 6 méthoxyhexahydro-4,7-méthanoindane-1 ou 2-carboxyaldéhyde ; le 3,7-diméthyl-octan-1-al, le 1-undécanal, le 10-undécén-1-al, le 4-hydroxy-3-méthoxybenzaldéhyde, le 1-méthyl-3-(4-méthylpentyl)-3-cyclohexène-carboxyaldéhyde, le 7-hydroxy-3,7-diméthyloctanal ; le trans-4décénal, le 2,6-nonadiénal, le para-tolylacétaldéhyde; le 4-méthylphénylacétaldéhyde, le 2-méthyl-4-(2,6,6-triméthyl-1-cyclo-hexén-1-yl)-2-buténal, l'ortho-méthoxycinnamaldéhyde, le S.S.δ-triméthyl-S-cyclohexènecarboxaldéhyde ; le 3,7-diméthyl-2-méthylène-6-octénal, le phénoxyacétaldéhyde ; le 5,9-diméthyl-4,8-décadiénal, le Peony aldéhyde (6,10-diméthyl-3-oxa-5,9-undécadién-1-al), l'hexahydro4,7-méthanoindane-1-carboxaldéhyde, le 2-méthyloctanal, l'alpha-méthyl-4-(I-méthyléthyl)benzène-acétaldéhyde, le 6,6-diméthyl-2-norpinène-2-propionaldéhyde, le para-méthylphénoxyacétaldéhyde ; le 2-méthyl-3-phényl-2-propén-1-al, le 3,5,5-triméthylhexanal, l'hexahydro-8,8-diméthyl-2-naphtaldéhyde, le 3-propyl-bicyclo-[2.2.1]-hept-5-ène-2-carbaldéhyde, le 9-décénal, le 3-méthyl-5-phényl-1-pentanal, le méthylnonylacétaldéhyde, le 1-p-menthène-q-carboxaldéhyde, le citral, le lilial, le 1-décanal, le florhydral et le 2,4-diméthyl-3-cyclohexène-1-carboxaldéhyde.

5. Agent de lavage ou de nettoyage selon l'une quelconque des revendications 1 à 4, **caractérisé en ce que**, par exposition à un rayonnement électromagnétique comprenant les longueurs d'onde de 200 à 400 nm, est libérée au moins une cétone faisant office de parfum, choisie en particulier parmi le groupe comprenant la buccoxime ; l'isojasmone ; la méthyl-β-naphtylcétone ; la Moschus indanone ; le Tonalide/Moschus plus ; l'alpha-damascone, la bêta-damascone, la delta-damascone, l'iso-damascone, la damascénone, la damarose, le dihydrojasmonate de méthyle, la menthone, la carvone, le camphre, la fenchone, l'alpha-ionène, la bêta-ionone, la di-hydro-bêta-ionone, l'ionone dénommée gamma-méthyle, la fleuramone, la dihydrojasmone, la cis-jasmone, l'iso-E-Super, la méthyl-cédrényl-cétone ou le méthyl-cédrylone, l'acetophénone, la méthyl-acétophénone, la para-méthoxy-acétophénone, la méthyl-bêta-naphtyl-cétone, la benzyl-acétone, la benzophénone, la para-hydroxy-phényl-butanone, la cétone du céleri ou livescone, la 6-isopropyldécahydro-2-naphtone, la diméthylocténone, la freskomenthe, la 4-(I-éthoxyvinyl)-3,3,5,5,-tétraméthyl-cyclohexanone, la méthyl-hepténone, la 2-(2(4-méthyl-3-cyclohexén-1-yl)propyl)-cyclopentanone, la 1-(p-menthén-6(2)-yl)-1-propanone, la 4-(4-hydroxy-3-méthoxyphényl)-2-butanone, le 2-acétyl-3,3-diméthyl-norbornane, la 6,7-dihydro-1,1,2,3,3-pentaméthyl-4(5H)-indanone, le 4-damascol, le dulcinyle ou la cassione, le gelsone, l'hexalone, l'isocyclémone E, la méthyl-cyclocitrone, la méthyl-lavande-cétone, l'orivone, la para-tert-butyl-cyclohexanone, la verdone, le delphone, la muscone, la néobuténone, la plicatone, la veloutone, la 2,4,4,7-tétraméthyl-oct-6-én-3-one, le tétramérane et l'hédione.

6. Agent de lavage ou de nettoyage selon l'une quelconque des revendications 1 à 5, **caractérisé en ce qu'**il contient au moins une des β-hydroxycétones répondant aux formules (VI)-(VII) :

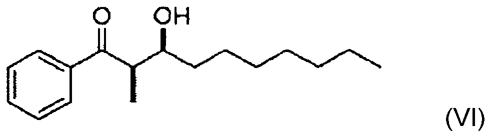

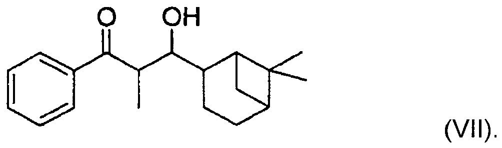

7. Agent de lavage ou de nettoyage selon l'une quelconque des revendications 1 à 6, **caractérisé en ce qu'**il contient au moins un agent tensioactif choisi parmi le groupe constitué par des agents tensioactifs anioniques, cationiques, non ioniques, zwitterioniques, amphotères, ou des mélanges desdits agents tensioactifs.

8. Procédé pour la libération de composés carbonyle répondant à la formule (V) 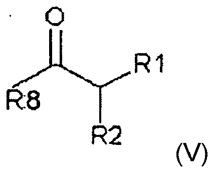 à partir de β-hydroxycétones répondant à la formule générale (I) 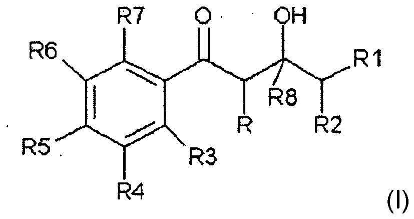 par exposition à un rayonnement électromagnétique comprenant les longueurs d'ondes de 200 à 400 nm, dans lequel, dans cette formule, R et R8 représentent, indépendamment l'un de l'autre, un atome d'hydrogène ou un résidu organique, en particulier un groupe alkyle ou alkylène linéaire ou ramifié,
substitué ou non substitué contenant de 1 à 20 atomes de carbone, mais de préférence un résidu méthyle,
et dans lequel R1 et R2, représentent, indépendamment l'un de l'autre, un atome d'hydrogène ou des résidus organiques par exemple en particulier un groupe alkyle ou un groupe alkylène linéaire ou ramifié, substitué ou non substitué, contenant de 1 à 20 atomes de carbone, de préférence de 4 à 20 atomes de carbone, les résidus R1 et R2 pouvant également former ensemble un système nucléaire, en particulier un groupe monocycloalkyle ou un groupe polycycloalkyle non substitué ou substitué contenant de 3 à 8 atomes de carbone ou un groupe phényle non substitué ou substitué,
et dans lequel R3, R4, R5, R6 et R7 représentent, indépendamment l'un de l'autre, un atome d'hydrogène, un atome d'halogène, un groupe NO₂, un groupe alcoxy linéaire ou ramifié, substitué ou non substitué contenant de 1 à 15 atomes de carbone ou un groupe alkyle linéaire ou ramifié, substitué ou non substitué contenant de 1 à 15 atomes de carbone.

9. Procédé selon la revendication 8, pour la libération d'aldéhydes faisant office de parfums, choisis en particulier parmi le groupe comprenant le mélonal, le triplal, le ligustral, l'adoxal; l'anisaldéhyde; le cymal; l'éthylvanilline ; le florhydral ; l'hélional ; l'héliotropine ; l'hydroxycitronellal ; la koavone ; le laurinaldéhyde ; le lyral ; le méthyl-nonyl-acétaldéhyde ; le p,t-bucinal ; le phénylacétaldéhyde ; le undécylène-aldéhyde ; la vanilline ; le 2,6,10-triméthyl-9-undécénal, le 3-dodécén-1-al, l'alpha-n-amyl-cinnamaldéhyde, le 4-méthoxybenzaldéhyde, le benzaldéhyde, le 3-(4-tert-butylphényl)-propanal, le 2-méthyl-3-(para-méthoxy-phénylpropanal), le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzyaldéhyde, le 1,2,3,4,5,6,7,8-octahydro-8,8-diméthyl-2-naphtaldéhyde, le 2,4-diméthyl-3-cyclohexén-1-carboxyaldéhyde, le 2-méthyl-3-(isopropylphényl)propanal, le décylaldéhyde, le 2,6-diméthyl-5-hepténal ; le 4-(tricyclo[5.2.1.0(2,6)]-décylidène-8)-butanal ; l'octahydro-J-méthano-IH-indènecarboxaldéhyde ; le 3-éthoxy-4-hydroxybenzaldéhyde, le para-éthyl-alpha,alpha-diméthylhydro-cinnamaldéhyde, l'alpha-méthyl-3,4-(méthylènedioxy)-hydrocinnamaldéhyde, le 3,4-méthylènedioxybenzaldéhyde, l'alpha-n-hexylcinnamaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexènecarboxaldéhyde, le 1-dodécanal, le 2,4-diméthylcyclohexène-S-carboxaldéhyde, le 4-(4-hydroxy-4-méthylpentyl-S-cylohexène-1-carboxaldéhyde, le 7-méthoxy-3,7-diméthyl-octan-1-al, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5 ou 6 méthoxyhexahydro-4,7-méthanoindane-1 ou 2-carboxyaldéhyde ; le 3,7-diméthyl-octan-1-al, le 1-undécanal, le 10-undécén-1-al, le 4-hydroxy-3-méthoxybenzaldéhyde, le 1-méthyl-3-(4-méthylpentyl)-3-cyclohexène-carboxyaldéhyde, le 7-hydroxy-3,7-diméthyloctanal ; le trans-4décénal, le 2,6-nonadiénal, le para-tolylacétaldéhyde ; le 4-méthylphénylacétaldéhyde, le 2-méthyl-4-(2,6,6-triméthyl-1-cyclo-hexén-1-yl)-2-buténal, l'ortho-méthoxycinnamaldéhyde, le S.S.δ-triméthyl-S-cyclohexènecarboxaldéhyde ; le 3,7-diméthyl-2-méthylène-6-octénal, le phénoxyacétaldéhyde ; le 5,9-diméthyl-4,8-décadiénal, le Peony aldéhyde (6,10-diméthyl-3-oxa-5,9-undécadién-1-al), l'hexahydro4,7-méthanoindane-1-carboxaldéhyde, le 2-méthyloctanal, l'alpha-méthyl-4-(I-méthyléthyl)benzène-acétaldéhyde, le 6,6-diméthyl-2-norpinène-2-propionaldéhyde, le para-méthylphénoxyacétaldéhyde ; le 2-méthyl-3-phényl-2-propén-1-al, le 3,5,5-triméthylhexanal, l'hexahydro-8,8-diméthyl-2-naphtaldéhyde, le 3-propyl-bicyclo-[2.2.1]-hept-5-ène-2-carbaldéhyde, le 9-décénal, le 3-méthyl-5-phényl-1-pentanal, le méthylnonylacétaldéhyde, le 1-p-menthène-q-carboxaldéhyde, le citral, le lilial, le 1-décanal, le florhydral et le 2,4-diméthyl-3-cyclohexène-1-carboxaldéhyde.

10. Procédé selon la revendication 8, pour la libération de cétones faisant office de parfums, choisies en particulier parmi le groupe comprenant la buccoxime ; l'isojasmone ; la méthyl-β-naphtylcétone ; la Moschus indanone ; le Tonalide/Moschus plus ; l'alpha-damascone, la bêta-damascone, la delta-damascone, l'iso-damascone, la damascénone, la damarose, le dihydrojasmonate de méthyle, la menthone, la carvone, le camphre, la fenchone, l'alpha-ionène, la bêta-ionone, la di-hydro-bêta-ionone, l'ionone dénommée gamma-méthyle, la fleuramone, la dihydrojasmone, la cis-jasmone, l'iso-E-Super, la méthyl-cédrényl-cétone ou le méthyl-cédrylone, l'acetophénone, la méthyl-acétophénone, la para-méthoxy-acétophénone, la méthyl-bêta-naphtyl-cétone, la benzyl-acétone, la benzophénone, la para-hydroxy-phényl-butanone, la cétone du céleri ou livescone, la 6-isopropyldécahydro-2-naphtone, la diméthylocténone, la freskomenthe, la 4-(I-éthoxyvinyl)-3,3,5,5,-tétraméthyl-cyclohexanone, la méthyl-hepténone, la 2-(2(4-méthyl-3-cyclohexén-1-yl)propyl)-cyclopentanone, la 1-(p-menthén-6(2)-yl)-1-propanone, la 4-(4-hydroxy-3-méthoxyphényl)-2-butanone, le 2-acétyl-3,3-diméthyl-norbornane, la 6,7-dihydro-1,1,2,3,3-pentaméthyl-4(5H)-indanone, le 4-damascol, le dulcinyle ou la cassione, le gelsone, l'hexalone, l'isocyclémone E, la méthyl-cyclocitrone, la méthyl-lavande-cétone, l'orivone, la para-tert-butyl-cyclohexanone, la verdone, le delphone, la muscone, la néobuténone, la plicatone, la veloutone, la 2,4,4,7-tétraméthyl-oct-6-én-3-one, le tétramérane et l'hédione.

11. Procédé pour parfumer des surfaces de manière durable, **caractérisé en ce qu'**on applique une composition selon l'une quelconque des revendications 1 à 7, sur une surface à parfumer et on expose ensuite la surface mentionnée à un rayonnement électromagnétique comprenant les longueurs d'ondes de 200 à 400 nm.

12. Utilisation de β-hydroxycétones répondant à la formule générale (I) 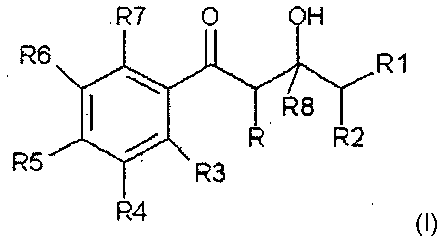 à titre de précurseurs de parfums dans des agents de lavage et de nettoyage, dans laquelle, dans cette formule, R et R8 représentent, indépendamment l'un de l'autre, un atome d'hydrogène ou un résidu organique, en particulier un groupe alkyle ou alkylène linéaire ou ramifié, substitué ou non substitué contenant de 1 à 20 atomes de carbone, mais de préférence un résidu méthyle, et dans laquelle R1 et R2, représentent, indépendamment l'un de l'autre, un atome d'hydrogène ou des résidus organiques par exemple en particulier un groupe alkyle ou un groupe alkylène linéaire ou ramifié, substitué ou non substitué, contenant de 1 à 20 atomes de carbone, de préférence de 4 à 20 atomes de carbone, les résidus R1 et R2 pouvant également former ensemble un système nucléaire, en particulier un groupe monocycloalkyle ou un groupe polycycloalkyle non substitué ou substitué contenant de 3 à 8 atomes de carbone ou un groupe phényle non substitué ou substitué,
et dans laquelle R3, R4, R5, R6 et R7 représentent, indépendamment l'un de l'autre, un atome d'hydrogène, un atome d'halogène, un groupe NO₂, un groupe alcoxy linéaire ou ramifié, substitué ou non substitué contenant de 1 à 15 atomes de carbone ou un groupe alkyle linéaire ou ramifié, substitué ou non substitué contenant de 1 à 15 atomes de carbone.

13. Utilisation de β-hydroxycétones répondant à la formule générale (I) 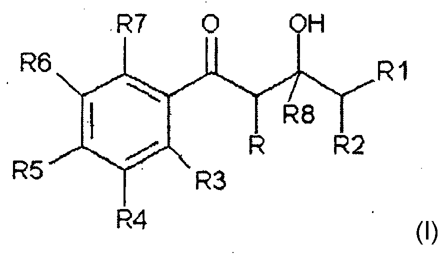 à titre de précurseurs de parfums dans des agents cosmétiques, dans laquelle, dans cette formule, R et R8 représentent, indépendamment l'un de l'autre, un atome d'hydrogène ou un résidu organique, en particulier un groupe alkyle ou alkylène linéaire ou ramifié, substitué ou non substitué contenant de 1 à 20 atomes de carbone, mais de préférence un résidu méthyle,
et dans laquelle R1 et R2, représentent, indépendamment l'un de l'autre, un atome d'hydrogène ou des résidus organiques par exemple en particulier un groupe alkyle ou un groupe alkylène linéaire ou ramifié, substitué ou non substitué, contenant de 1 à 20 atomes de carbone, de préférence de 4 à 20 atomes de carbone, les résidus R1 et R2 pouvant également former ensemble un système nucléaire, en particulier un groupe monocycloalkyle ou un groupe polycycloalkyle non substitué ou substitué contenant de 3 à 8 atomes de carbone ou un groupe phényle non substitué ou substitué,
et dans laquelle R3, R4, R5, R6 et R7 représentent, indépendamment l'un de l'autre, un atome d'hydrogène, un atome d'halogène, un groupe NO₂, un groupe alcoxy linéaire ou ramifié, substitué ou non substitué contenant de 1 à 15 atomes de carbone ou un groupe alkyle linéaire ou ramifié, substitué ou non substitué contenant de 1 à 15 atomes de carbone.

14. Utilisation de β-hydroxycétones répondant à la formule générale (I) 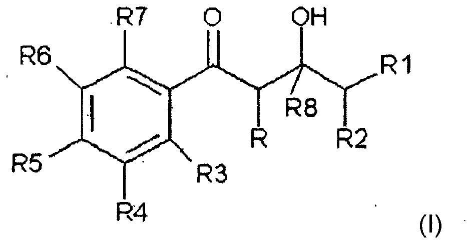 à titre de précurseurs de parfums dans des désodorisants, dans laquelle, dans cette formule, R et R8 représentent, indépendamment l'un de l'autre, un atome d'hydrogène ou un résidu organique, en particulier un groupe alkyle ou alkylène linéaire ou ramifié, substitué ou non substitué contenant de 1 à 20 atomes de carbone, mais de préférence un résidu méthyle,
et dans laquelle R1 et R2, représentent, indépendamment l'un de l'autre, un atome d'hydrogène ou des résidus organiques par exemple en particulier un groupe alkyle ou un groupe alkylène linéaire ou ramifié, substitué ou non substitué, contenant de 1 à 20 atomes de carbone, de préférence de 4 à 20 atomes de carbone, les résidus R1 et R2 pouvant également former ensemble un système nucléaire, en particulier un groupe monocycloalkyle ou un groupe polycycloalkyle non substitué ou substitué contenant de 3 à 8 atomes de carbone ou un groupe phényle non substitué ou substitué,
et dans laquelle R3, R4, R5, R6 et R7 représentent, indépendamment l'un de l'autre, un atome d'hydrogène, un atome d'halogène, un groupe NO₂, un groupe alcoxy linéaire ou ramifié, substitué ou non substitué contenant de 1 à 15 atomes de carbone ou un groupe alkyle linéaire ou ramifié, substitué ou non substitué contenant de 1 à 15 atomes de carbone.
